# EUROPEAN PATENT SPECIFICATION

(11) **EP 1 582 141 B1**
(45) Date of publication and mention of the grant of the patent: **18.05.2011**
(21) Application number: 05251998.0
(22) Date of filing: 30.03.2005
(51) Int. Cl.: A61B 1/12, A61M 25/10

(54) **Balloon control apparatus**
Steuerungsvorrichtung für einen Ballon
Appareil de contrôle d'un ballon

(30) Priority: 31.03.2004 JP 2004107180; 04.11.2004 JP 2004321221
(43) Date of publication of application: 05.10.2005
(73) Proprietor: FUJIFILM Corporation, Minato-ku Tokyo 106-8620 (JP); SRJ Corporation, Kawachi-gun Tochigi (JP)
(72) Inventor: Sekiguchi, Tadashi c/o Fujinon Corporation, Saitama-shi, Saitama (JP)
(74) Representative: Stevens, Jason Paul

(56) References cited:
- WO-A-03/105563
- US-A1- 2002 072 647
- US-A1- 2003 004 460
- US-B1- 6 547 723

## Description

The present invention relates to a balloon control apparatus and, more particularly, to a balloon control apparatus for use with a medical endoscope apparatus for observing a deep-part digestive tract such as the small intestine or the large intestine.

When an insertion portion of an endoscope is inserted into a deep-part digestive tract such as the small intestine, a force for insertion cannot be easily transmitted to the tip of the insertion portion due to the existence of complicated bends in the intestinal tract and it is difficult to insert the insertion portion to a deep part, if the insertion portion is simply forced into the tract. For example, if an unnecessary bend or warp is caused in the insertion portion, the insertion portion cannot be inserted to a deeper portion of the tract. A method has therefore been proposed in which the insertion portion of an endoscope is inserted into a body cavity together with an insertion assist implement which caps the insertion portion, and an unnecessary bend or warp in the insertion portion is prevented by guiding the insertion portion with the insertion assist implement.

Japanese Patent Application Laid Open No. 51-11689 discloses an endoscope apparatus in which a first balloon is provided on an insertion portion of an endoscope close to the tip of the insertion portion and a second balloon is provided on an insertion assist implement (also referred to as an over tube or sliding tube) close to the tip of the insertion assist implement. This endoscope apparatus is capable of inserting the insertion portion to a deep portion of an intestinal tract such as the small intestine having complicated bends by alternately inserting the insertion portion and the insertion assist implement while repeating expanding and contracting the first and second balloons.

In the conventional endoscope apparatus, operating buttons are provided in correspondence with operations for expansion and contraction of the first balloon and operations for expansion and contraction of the second balloon. An operator selects one of the plurality of operating buttons and operates the selected operating button. If the operation procedure is complicated, there is a risk of the operator making an error in operating the operating buttons. Further, there is a problem that the operator pays so much attention to the operation of the plurality of operating buttons that he or she cannot concentrate on operating the endoscope.

Colonoscopes with program controls for the balloons are disclosed in DE 3631352 and DE 3630660. In addition, US 4690131 discloses an endoscope where the inflation and deflation of balloons can be controlled by a camming system. However, these control systems do not allow an operator to switch between manual and automatic operation.

In view of the above-described circumstances, an object of at least the preferred embodiments of the present invention is to provide an endoscope balloon control apparatus which enables simplification of the balloon expansion/contraction operation.

According to a first aspect of the present invention, there is provided a balloon control apparatus which controls operations for expanding and contracting a first balloon attached to an insertion portion of an endoscope, and operations for expanding and contracting a second balloon attached to an insertion assist implement fitted around the insertion portion to assist insertion of the insertion portion, the apparatus characterized by comprising:
a storage device which stores an operating procedure indicating a sequence of the operation for expanding the first balloon, the operation for contracting the first balloon, the operation for expanding the second balloon, and the operation for contracting the second balloon;
an execution direction device which directs execution of the expanding and contracting operations according to the operation procedure stored by the storage device;
a plurality of manual operation buttons individually corresponding to the operation for expanding the first balloon, the operation for contracting the first balloon, the operation for expanding the second balloon, and the operation for contracting the second balloon;
an automatic operation button corresponding to the execution direction device; and
a mode change switch which selects between a manual mode in which the manual operation buttons are made operable and an automatic mode in which the automatic operation button is made operable,
wherein a direction is given by the execution direction device to execute the operation for expanding the first balloon, the operation for contracting the first balloon, the operation for expanding the second balloon, and the operation for contracting the second balloon in accordance with the sequence indicated by the operating procedure stored by the storage device.

According to this aspect of the present invention, the balloon expanding and contracting operations are executed in accordance with the operating procedure each time a direction is given from the execution direction device. Therefore, the operator can easily perform the operations even in a case where the operating procedure is complicated. As a result, the operator can concentrate on operating the endoscope.

In a preferred form, the automatic mode is automatically cancelled when one of the manual operation buttons is operated.

It is further preferred for the apparatus to further include a learning function which stores in the storage device the sequence of operations defined by operation of the manual operation buttons as the operating procedure.

In a further preferred form, the storage device stores a plurality of operating procedures each of which has a different sequence of the operation for expanding the first balloon, the operation for contracting the first balloon, the operation for expanding the second balloon, and the operation for contracting the second balloon, and
the apparatus further comprises a selection operation device which selects an operating procedure to be executed from among the plurality of operating procedures.

In the balloon control apparatus in accordance with the present invention, the balloon expanding and contracting operations are executed in accordance with the operating procedure each time a direction is given from the execution direction device. Therefore, the operator may only operate the operation buttons and can concentrate on operating the endoscope.

Preferred embodiments of the invention will now be described by way of example only and with reference to the accompanying drawings, in which:
Fig. 1 is a perspective view of equipment in an examination room to which a balloon control apparatus in accordance with the present invention is applied;
Fig. 2 is a diagram showing the configuration of a system representing an endoscope apparatus to which the balloon control apparatus in accordance with the present invention is applied;
Fig. 3 is a perspective view showing a fore end of the insertion portion shown in Fig. 2;
Fig. 4 is a side view of the construction of the holding device shown in Fig. 1;
Fig. 5 is a sectional view taking along the line 5-5 in Fig. 4;
Fig. 6 is a sectional view taking along the line 6-6 in Fig. 4;
Fig. 7 is a perspective view of the configuration of the balloon control apparatus;
Fig. 8 is a block diagram showing the internal configuration of the balloon control apparatus;
Fig. 9 is a flowchart showing an operating procedure for the endoscope apparatus in the embodiment of the present invention;
Figs. 10A to 10H are diagrams showing the operating procedure for the endoscope apparatus in the embodiment of the present invention;
Fig. 11 is a flowchart showing an operating procedure different from that shown in Fig. 9;
Fig. 12 is a flowchart showing an operating procedure different from that shown in Fig. 9;
Fig. 13 is a flowchart showing an operating procedure different from that shown in Fig. 9;
Fig. 14 is a flowchart showing an operating procedure different from that shown in Fig. 9;
Fig. 15 is a flowchart showing an operating procedure at the time of drawing out;
Fig. 16 is a flowchart showing an operating procedure different from that shown in Fig. 15;
Fig. 17 is a flowchart showing an operating procedure different from that shown in Fig. 15;
Fig. 18 is a flowchart showing an operating procedure different from that shown in Fig. 15;
Fig. 19 is a perspective view of another example of the holding device;
Fig. 20 is a plan view of the holding device shown in Fig. 19;
Fig. 21 is a plan view of the holding device holding the at-hand operating portion of the endoscope;
Fig. 22 is a plan view of the holding device holding the base end portion of the insertion assist implement;
Fig. 23 is a perspective view of a holding device having a structure different from that shown in Fig. 19;
Fig. 24 is a perspective view of a modification of the holding device shown in Fig. 23:
Fig. 25 is a perspective view of a holding device having a structure different from that shown in Fig. 19;
Fig. 26 is a perspective view of another example of the holding device;
Fig. 27 is a perspective view of a further example of the holding device;
Fig. 28 is a front view of a holding device having a structure different from that shown in Fig. 5;
Fig. 29 is a front view of a holding device having a structure different from that shown in Fig. 5; and
Fig. 30 is a front view of a holding device having a structure different from that shown in Fig. 5.

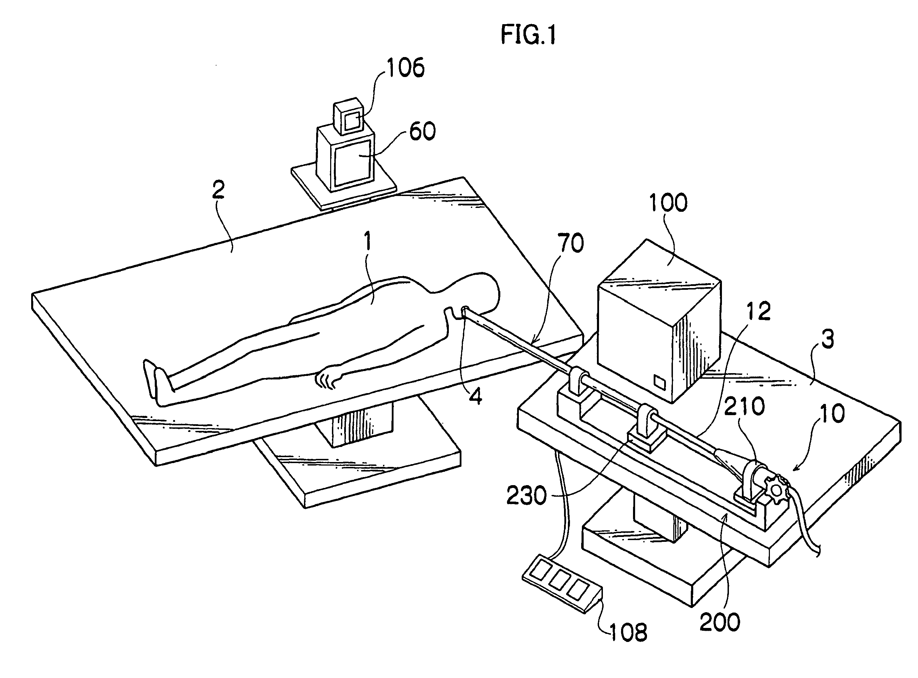

Fig. 1 is a perspective view of equipment in an examination room to which a balloon control apparatus in accordance with the present invention is applied. As shown in Fig. 1, an examination table 2 on which a patient 1 is laid is provided in the examination room. An operator (not shown) performs an operation while standing on the front side of the examination table 2. A monitor 60 and a balloon monitor 106 described below as a monitor specially used for observing the states of balloons are provided on the inner side of the examination table 2.

An assist table 3 is placed adjacent to the examination table 2 on the front side of the examination table 2. A holding device 200 described below is mounted on the assist table 3, and an endoscope 10 and an insertion assist implement 70 are held by the holding device 200. Other units described below, including a light source unit 20, a processor 30 and a balloon control apparatus 100, may be mounted on the assist table 3. A larger examination table 2 may be provided and the holding device 200 may be mounted on the larger examination table 2 instead of being mounted on the assist table 3.

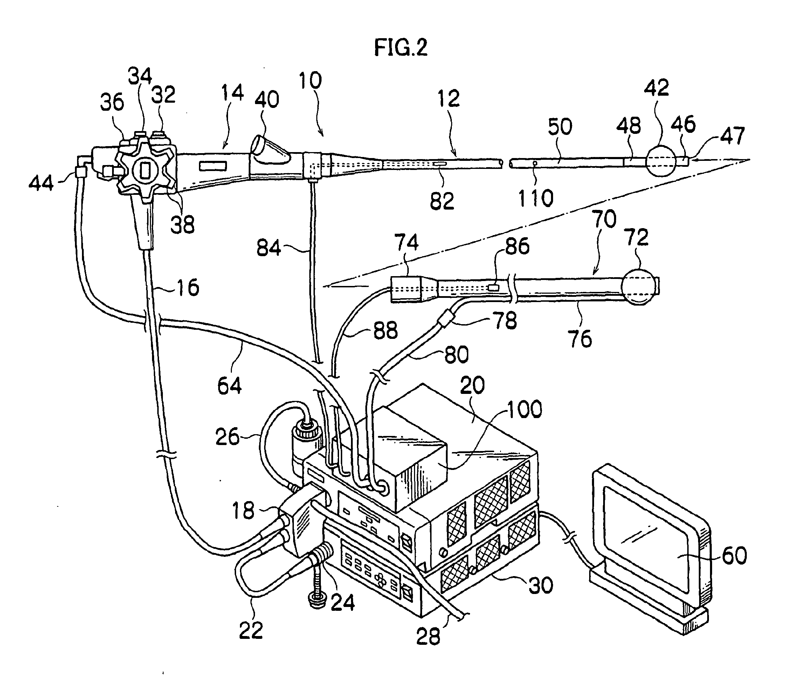

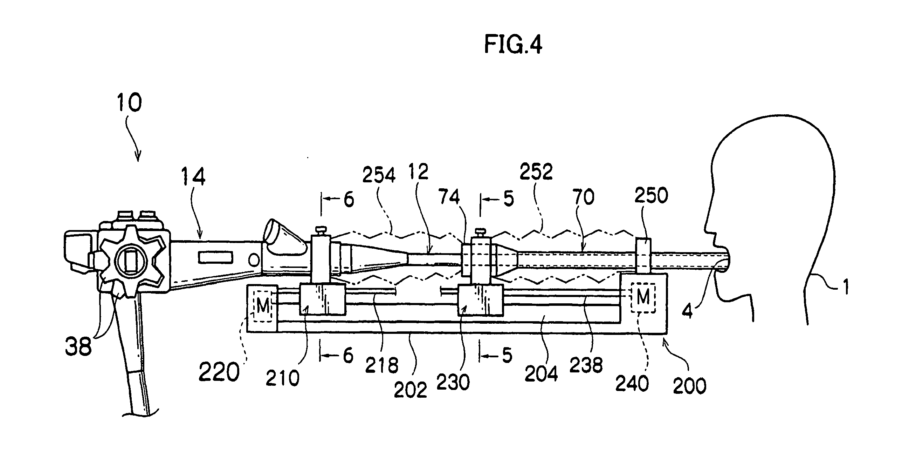

Fig. 2 is a diagram showing the configuration of a system for an implementation of an endoscope apparatus using the balloon control apparatus in accordance with the present invention. The endoscope apparatus in this implementation is constituted mainly by the endoscope 10, the light source unit 20, the processor 30, the insertion assist implement 70, the balloon control apparatus 100 and the below-described holding device 200 shown in Fig. 4.

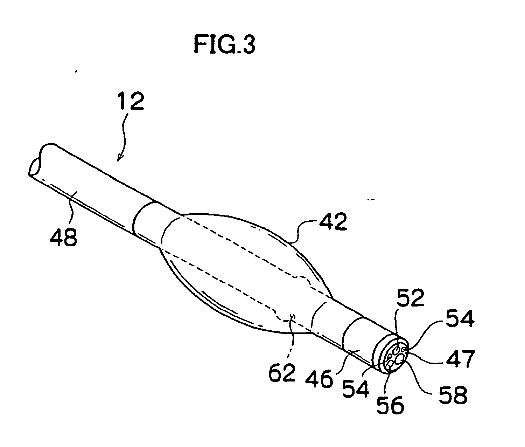

As shown in Fig. 2, the endoscope 10 has an insertion portion 12, which is inserted into a body cavity, and an at-hand operating portion 14 joined to the insertion portion 12. One end of a universal cable 16 is connected to the at-hand operating portion 14, and an LG connector 18 is provided at the other end of the universal cable 16. The LG connector 18 is detachably attached to the light source unit 20 to enable transmission of illumination light to an illumination optical system 54 described below (see Fig. 3). An electric connector 24 is connected to the LG connector 18 via a cable 22 and is detachably attached to the processor 30. The LG connector 18 is connected to a water tank 27 via a tube 26 for supply of air and water to enable supply of water in the water tank 27. The LG connector 18 is also connected to an aspiration unit (not shown) via an aspiration tube 28 to enable inhalation of air through the tube 28.

The insertion portion 12 includes a foremost portion 46, a bending portion 48 and a soft portion 50. The bending portion 48 is remotely operated by rotating a pair of angle knobs 38 provided on the at-hand operating portion 14 so that the bending portion 48 bends. In this way, a foremost end surface 47 of the foremost end portion 46 can be directed as desired.

As shown in Fig. 3, an observation optical system 52, illumination optical systems 54, an air/water supply nozzle 56 and a forceps opening 58 are provided in the foremost end surface 47 of the foremost end portion 46. A charge-coupled device (CCD) (not shown) is provided at the rear of the observation optical system 52, and a signal cable is connected to a base plate on which the CCD is supported. The signal cable is extended to the electric connector 24 by being passed through the insertion portion 12, the at-hand operating portion 14 and the universal cable 16 shown in Fig. 2 to be connected to the processor 30. An observed image taken into the observation optical system 52 is received through the light receiving surface of the CCD and converted into an electrical signal. This electrical signal is output to the processor 30 via the signal cable to be converted into a video signal, thus enabling the observed image to be displayed on the monitor 60 connected to the processor 30.

At the rear of the illumination optical systems 54 shown in Fig. 3, an emergence end of a light guide (not shown) is provided. This light guide passes through the insertion portion 12, the at-hand operating portion 14 and the universal cable 16 shown in Fig. 2 and has its incidence end placed in the LG connector 18. The LG connector 18 is connected to the light source unit 20 to enable illumination light emitted from the light source unit 20 to be transmitted to the illumination optical systems 54 through the light guide and radiated forward from the illumination optical systems 54.

The air/water supply nozzle 56 shown in Fig. 3 communicates with a valve (not shown) operated with an air/water supply button 32 shown in Fig. 2. This valve is connected to the tank 27 via the air/water supply tube 26 and is also connected to an air pump (not shown) in the light source unit 20. The air/water supply button 32 is operated to supply air from the air pump or water from the tank 27 to the air/water supply nozzle 56. Air or water thus supplied can be jetted through the air/water supply nozzle 56 toward the observation optical system 52.

The forceps opening 58 shown in Fig. 3 communicates with a forceps insertion portion 40 shown in Fig. 2. An implement for a treatment can be inserted into the endoscope through the forceps insertion portion 40 and guided to the forceps opening 58 to project from the same. The forceps opening 58 also communicates with a valve (not shown) operated with an aspiration button 34. This valve communicates with the aspiration tube 28. A lesion portion or the like can be sucked through the forceps opening 58 by driving an aspiration device connected to an end of the aspiration tube 28.

As shown in Fig. 3, a first balloon 42 made of an elastic material such as rubber is fitted around the peripheral surface of the insertion portion 12. The first balloon 42 is formed into a generally cylindrical shape constricted at its opposite ends. The first balloon 42 is attached to the insertion portion 12 in such a manner that the insertion portion 12 is passed through the first balloon 42 and placed in a desired position and opposite end portions of the first balloon 42 are thereafter fixed on the insertion portion 12.

An air hole 62 is formed in the peripheral surface of the insertion portion 12 at a position corresponding to the attached position of the first balloon 42. The air hole 62 communicates with a supply/aspiration port 44 shown in Fig. 2 through a tube (not shown). A tube 64 is connected between the supply/aspiration port 44 and the balloon control apparatus 100. The balloon control apparatus 100 is an apparatus for supplying air to the first balloon 42 through the tube 64, inhaling air from the first balloon 42 and controlling the air pressure during supply or inhalation of air. When air is supplied to the first balloon 42, the first balloon 42 expends so as to be generally spherical. When air is inhaled from the first balloon 42, the first balloon 42 adheres to the outer surface of the insertion portion 12.

The insertion assist implement 70 shown in Fig. 2 is formed into a cylindrical shape, has an inside diameter slightly larger than the outside diameter of the insertion portion 12, and has sufficiently high flexibility. The insertion assist implement 70 has a hard hold portion 74 at its base end. The insertion portion 12 is inserted into the insertion assist implement 70 through the hold portion 74. A second balloon 72 made of latex is attached to the insertion assist implement 70 in the vicinity of the tip of the insertion assist implement 70. The second balloon 72 is formed into a generally cylindrical shape constricted at its opposite ends. The second balloon 72 is attached to the insertion assist implement 70 passed through the second balloon 72. A tube 76 attached to the peripheral surface of the insertion assist implement 70 communicates with the second balloon 72. A connector 78 is provided on an end portion of the tube 76, and a tube 80 is detachably connected to the connector 78. The tube 80 is connected to the balloon control apparatus 100. The balloon control apparatus 100 supplies air to the tube 80 or inhales air from the tube 80 and controls the air pressure during supply or inhalation of air. Air can be supplied to or inhaled from the second balloon 72 by driving the balloon control apparatus 100. When air is supplied to the second balloon 72, the second balloon 72 expends so as to be generally spherical. When air is inhaled from the second balloon 72, the second balloon 72 adheres to the outer surface of the insertion assist implement 70.

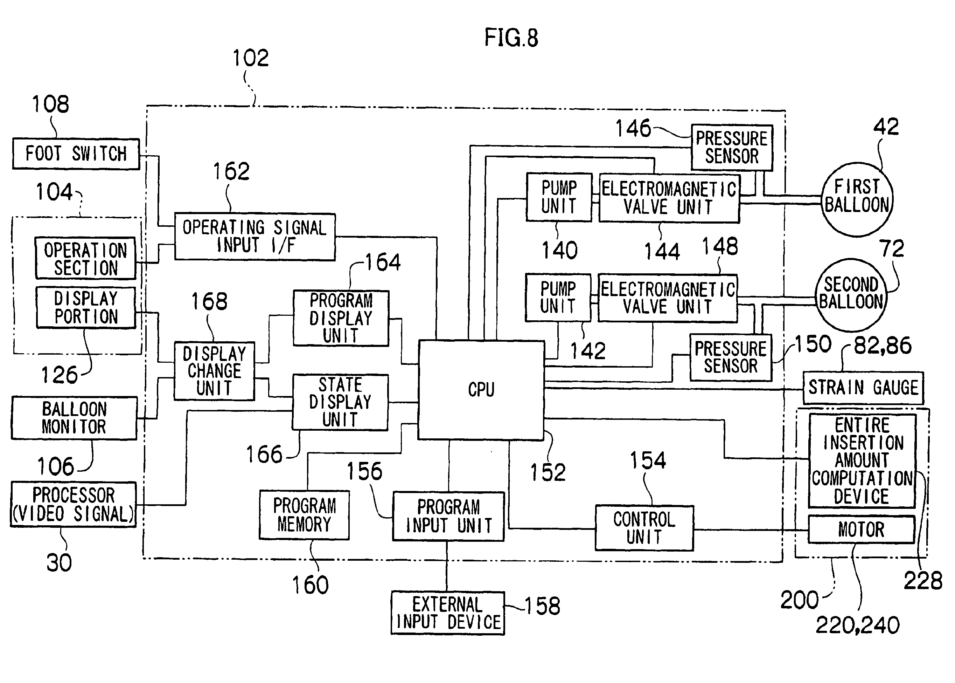

As shown in Fig. 2, a strain gauge 82 for measuring a force by which the insertion portion 12 is drawn out is provided at a predetermined position on the insertion portion 12. A signal line 84 is connected to the strain gauge 82. The signal line 84 is passed through the insertion portion 12, extended to the outside through the at-hand operating portion 14 and connected to the balloon control apparatus 100. When the electrical resistance value of the strain gauge 82 exceeds a threshold value, the balloon control apparatus 100 leaks air from the first balloon 42 by communication between the outside and an electromagnetic valve unit 144 (see Fig. 8) communicating with the first balloon 42. Accordingly, when the strain in the insertion portion 12 becomes large, the first balloon 42 contracts to reduce the drawing-out force, thereby preventing the drawing-out force from becoming larger than a set value. Therefore, the insertion portion 12 can be drawn out without putting a considerable burden on a patient.

On the other hand, a strain gauge 86 for measuring a force by which the insertion assist implement 70 is drawn out is provided at a predetermined position on the insertion assist implement 70. A signal line 88 is connected to the strain gauge 86. The signal line 88 is passed through the insertion assist implement 70, extended to the outside through the hold portion 74 and connected to the balloon control apparatus 100. When the electrical resistance value of the strain gauge 88 exceeds a threshold value, the balloon control apparatus 100 leaks air from the second balloon 72 by communication between the outside and an electromagnetic valve unit 148 (see Fig. 8) communicating with the second balloon 72. Accordingly, when the strain in the insertion assist implement 70 becomes large, the second balloon 72 contracts to reduce the drawing-out force. The drawing-out force is thereby prevented from becoming larger than a set value when the insertion portion 12 is drawn out from a patient. Therefore, the insertion portion 12 can be drawn out without putting a considerable burden on the patient.

The arrangement may be such that forcing-in forces by which the insertion portion 12 and the insertion assist implement 70 are inserted into a patient are measured with the above-described strain gauges 82 and 86 and forcing-in is controlled so that each forcing-in force does not exceed a threshold value. Thus, a load such as a drawing-out force or a forcing-in force is measured and the movement of the endoscope 10 or the insertion assist implement 70 is limited on the basis of the measured value to prevent imposition of a considerable burden on a patient. When the load becomes large during the movement of the endoscope 10 or the insertion assist implement 70, it is reduced by reducing the internal pressure in the first balloon 42 or the second balloon 72 by stopping movement of the endoscope 10 or the insertion assist implement 70 or by moving the endoscope 10 or the insertion assist implement 70 in the opposite direction. Also, the arrangement may be such that an automatic injection device for automatically injecting a lubricant between the inner circumferential surface of the insertion assist implement 70 and the outer circumferential surface of the insertion portion 12 is provided and the lubricant is supplied with reference to the load. That is, when the load becomes large, the rate of supply of the lubricant is increased to reduce the frictional resistance between the insertion assist implement 70 and the insertion portion 12, thereby reducing the load.

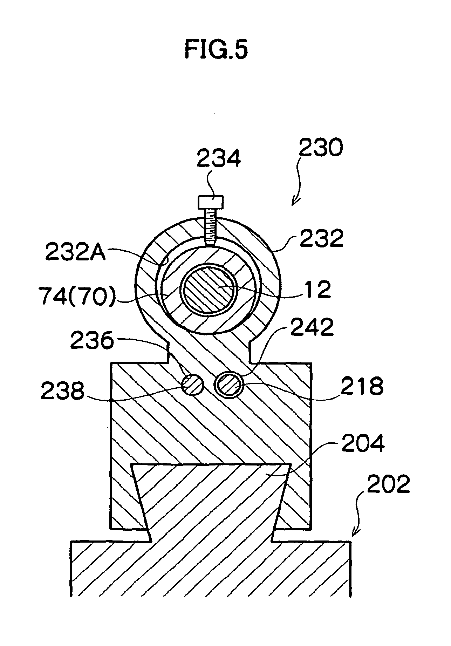

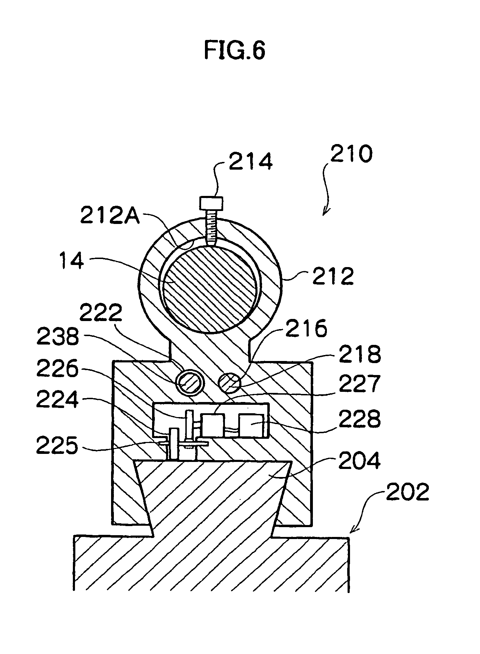

The endoscope 10 and the insertion assist implement 70 constructed as described above are held by the holding device 200 shown in Fig. 4. The holding device 200 has a stage 202 on which a guide rail 204 is laid. The guide rail 204 is placed along a straight line extending to the mouth 4 of the patient 1. An endoscope holder 210 and an assist implement holder 230 are slidably supported on the guide rail 204. The guide rail 204 is formed so as to be dovetailed as shown in Figs. 5 and 6. A dovetailed channel is formed in each of the endoscope holder 210 and the assist implement holder 230 to engage with the guide rail 204. Therefore the endoscope holder 210 and the assist implement holder 230 can slide along the guide rail 204 without coming off the same. The shapes for engagement between the guide rail 204 and the endoscope holder 210 or the assist implement holder 230 are not particularly specified and any shapes may suffice if the holders are slidably supported.

As shown in Fig. 5, the assist implement holder 230 has a ring portion 232, and a through hole 232A having an inside diameter slightly larger than the outside diameter of the hold portion 74 of the insertion assist implement 70 is formed in the ring portion 232. A fixing screw 234 is screwed into the ring portion 232 from the outer circumferential side. When the fixing screw 234 is driven, the tip of the fixing screw 234 projects from the inner circumferential surface of the ring portion 232. After the hold portion 74 of the insertion assist implement 70 has been inserted and placed in the through hole 232A of the ring portion 232, the fixing screw 234 is driven to bring the tip of the fixing screw 234 into engagement with the hold portion 74. The insertion assist implement 70 is thus held in the assist implement holder 230.

A screw hole 236 is formed in the assist implement holder 230, and a feed screw 238 is screwed into this screw hole 236. As shown in Fig. 4, the feed screw 238 is placed along the guide rail 204 and rotatably supported on the stage 202. A motor 240 for rotating the feed screw 238 is provided on an end portion of the stage 202. The feed screw 238 is rotated by the motor 240 to slide the assist implement holder 230 along the guide rail 204. That is, the assist implement holder 230 moves linearly relative to the mouth 4 of the patient 1. Thus, the insertion assist implement 70 held by the assist implement holder 230 can be linearly guided in movement relative to the mouth 4 of the patient 1.

Further, a hole 242 through which a feed screw 218 described below is passed is formed in the assist implement holder 230 shown in Fig. 5. This hole 242 is formed so as to be larger in outside diameter than the feed screw 218.

On the other hand, the endoscope holder 210 has a ring portion 212, and the ring portion 212 has a through hole 212A into which the at-hand operating portion 14 can be inserted. A fixing screw 214 is screwed into the ring portion 212 from the outer circumferential side. When the fixing screw 214 is driven, the tip of the fixing screw 214 projects from the inner circumferential surface of the ring portion 212. After the at-hand operating portion 14 has been inserted and placed in the through hole 212A, the fixing screw 214 is driven to bring the tip of the fixing screw 214 into engagement with the at-hand operating portion 14. The at-hand operating portion 14 is thereby held in the assist implement holder 230.

A screw hole 216 is formed in the endoscope holder 210, and the feed screw 218 is screwed into this screw hole 216. As shown in Fig. 4, the feed screw 218 is placed along the guide rail 204 and rotatably supported on the stage 202. A motor 220 for rotating the feed screw 218 is provided on an end portion of the stage 202. The feed screw 218 is rotated by the motor 220 to slide the endoscope holder 210 along the guide rail 204. That is, the endoscope holder 210 moves linearly relative to the mouth 4 of the patient 1. Thus, the insertion portion 12 of the endoscope 10 held by the endoscope holder 210 can be linearly guided in movement relative to the mouth 4 of the patient 1.

A hole 222 through which the above-mentioned feed screw 238 on the assist implement holder 230 side is passed is formed in the endoscope holder 210 shown in Fig. 6. This hole 222 is formed so as to be larger in outside diameter than the feed screw 238.

Further, a roller 224 provided as an insertion amount measurement device is rotatably supported on the endoscope holder 210. The roller 224 is placed so as to project on the guide rail 204 side and rotates by following the relative movement of the guide rail 204 when the endoscope holder 210 is slid along the guide rail 204.

A gear 226 is linked to a rotating shaft 225 of the roller 224 and a torque of the roller 224 is transmitted to the gear 226. The gear 226 is connected to a sensor 227 through a one-way clutch (not shown). The number of revolutions of the gear 226 in one direction only is detected by the sensor 227. The direction of revolutions detected is the direction in which the gear 226 rotates when the endoscope holder 210 is moved toward the mouth 4 of the patient 1.

A computation device 228 is connected to the sensor 227. The computation device 228 converts the number of revolutions of the gear 226 detected by the sensor 227 into the amount of insertion (length) of the insertion portion 12. The converted value is integrated to obtain the entire amount of insertion. The computation device 228 is connected to the balloon control apparatus 100 and the entire amount of insertion obtained by the computation device 228 is displayed on the balloon monitor 106 or the like in the balloon control apparatus 100, thereby enabling the operator to grasp to which position in a body cavity the fore end of the insertion portion 12 has reached.

As shown in Fig. 4, a guide ring 250 is provided on an end portion of the stage 202 on the fore end side. The guide ring 250 has an inside diameter slightly larger than the outside diameter of the insertion assist implement 70 such that the insertion assist implement 70 can be inserted into the guide ring 250 to be guided by the guide ring 250.

A cover 252 is provided between the guide ring 250 and the assist implement holder 230, as indicated by the double-dot-dash line. The cover 252 is formed into a cylindrical shape (e.g., a bellows-like shape) so as to be able to expand and contract easily and is attached so as to cover the insertion assist implement 70. Opposite end portions of the cover 252 are detachably connected to the guide ring 250 and the assist implement holder 230. The cover 252 can be detached and washed when necessary. The insertion assist implement 70 is covered with the cover 252 formed as described above to prevent a body fluid or the like attached to the outer surface of the insertion assist implement 70 from being scattered, thus enabling the operator to operate the endoscope without contaminating his or her hand.

A cover 254 is provided between the assist implement holder 230 and the endoscope holder 210, as indicated by the double-dot-dash line. The cover 254 is formed into a cylindrical shape (e.g., a bellows-like shape) so as to be able to expand and contract easily and is attached so as to cover the insertion portion 12, as is the cover 252. Opposite end portions of the cover 254 are detachably connected to the assist implement holder 230 and the endoscope holder 210. The cover 254 can be detached and washed when necessary. The insertion portion 12 is covered with the cover 254 formed as described above to prevent a body fluid or the like attached to the outer surface of the insertion portion 12 from being scattered, thus enabling the operator to operate the endoscope without contaminating his or her hand.

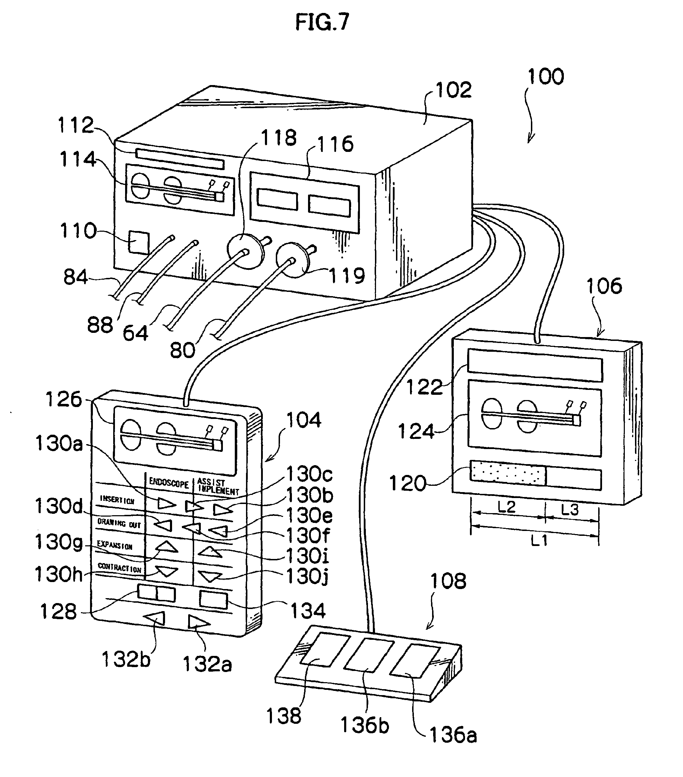

Fig. 7 is a perspective view of the balloon control apparatus 100. As shown in Fig. 7, the balloon control apparatus 100 is constituted mainly by a main unit 102, a remote controller 104, the balloon monitor 106 and a foot switch 108. A power switch 110, an error display portion 112, a state display portion 114, a pressure value display portion 116 and gas/liquid separation filters 118 and 119 are provided in or on a front panel of the main unit 102. The above-mentioned tubes 64 and 80 are connected to the gas/liquid separation filters 118 and 119. Liquids sucked through the tubes 64 and 80 are removed by gas/liquid separation in the gas/liquid separation filters 118 and 119. In this way, drawing of any liquid into the main unit 102 is prevented.

Characters, numerals or the like for expressing, for example, a state where the first balloon 42 or the second balloon 72 is burst are displayed on the error display portion 112. The states of expansion/contraction of the first balloon 42 and the second balloon 72 are shown on the state display portion 114. The internal pressures in the first and second balloons 41 and 72 measured with pressure sensors 146 and 150 (see Fig. 8) described below are shown on the pressure value display portion 116.

As shown in Fig. 1, the balloon monitor 106 is attached to the monitor 60 to enable the operator to observe through the screen of the balloon monitor 106 simultaneously with observation through screen of the monitor 60. An error display portion 122 and a state display portion 124 on which the same contents of information as those displayed on the error display portion 112 and the state display portion 114 are displayed are provided in the balloon monitor 106 shown in Fig. 7. It is, therefore, also possible to grasp the states of expansion/contraction of the first and second balloons 42 and 72 and occurrence of errors by monitoring through the balloon monitor 106. An entire insertion amount display portion 120 is also provided in the balloon monitor 106. In the entire insertion amount display portion 120, L2 indicating the entire amount of insertion is lighted in contrast with L1 indicating the entire length of the intestinal tract, thereby enabling L3 indicating the remaining length of the intestinal tract to be grasped at a look. The same display portion as the entire insertion amount display portion 120 may be provided in the front panel of the main unit 102 or in the remote controller 104.

In the remote controller 104, a state display portion 126 is provided on which the same display as that on the state display portion 114 is produced. The remote controller 104 is also provided with a mode change switch 128. The mode change switch 128 enables selection from a manual mode, an automatic mode and a learning mode. The remote controller 104 is further provided with operating buttons 130a to 130j (corresponding to the manual operation buttons) which are made operable when the manual mode or the learning mode (hereinafter referred to collectively as "manual/learning mode") is selected, operating buttons 132a and 132b (corresponding to the execution direction device and the automatic operation buttons) which are made operable when the automatic mode is selected, and a stop button 134 used in common in the different modes.

When the operating button 130a for operation in the manual/learning mode is operated by being depressed, the motor 220 shown in Fig. 4 is driven to move the endoscope holder 210 forward by a predetermined amount. Similarly, when the operating button 130b shown in Fig. 7 is operated by being depressed, the motor 240 shown in Fig. 4 is driven to move the assist implement holder 230 forward by a predetermined amount. When the operating button 130c shown in Fig. 7 is operated by being depressed, the endoscope holder 210 and the assist implement holder 230 shown in Fig. 4 are simultaneously moved forward by a predetermined amount.

When the operating button 130d shown in Fig. 7 is operated by being depressed, the motor 220 shown in Fig. 4 is driven to move the endoscope holder 210 rearward by a predetermined amount. Similarly, when the operating button 130e shown in Fig. 7 is operated by being depressed, the motor 240 shown in Fig. 4 is driven to move the assist implement holder 230 rearward by a predetermined amount. When the operating button 130f shown in Fig. 7 is operated by being depressed, the endoscope holder 210 and the assist implement holder 230 shown in Fig. 4 are simultaneously moved rearward by a predetermined amount.

When the operating button 130g shown in Fig. 7 is operated by being depressed, air is supplied to the first balloon 42 shown in Fig. 2. When the operating button 130h shown in Fig. 7 is operated by being depressed, air is inhaled from the first balloon 42 shown in Fig. 2. Similarly, when the operating button 130i shown in Fig. 7 is operated by being depressed, air is supplied to the second balloon 72 shown in Fig. 2. When the operating button 130j shown in Fig. 7 is operated by being depressed, air is inhaled from the second balloon 72 shown in Fig. 2.

When the operating button 132a for operation in the automatic mode is depressed one time, a transition to the next operation (step) is made. When the operating button 132b is operated by being depressed, a return to the preceding operation is made.

While only the state display portion 126 is provided in the above-described remote controller 104, other portions including an error display portion, an entire amount display portion and a pressure value display portion may also be provided in the remote controller 104. A mode indication portion for indicating the present mode may also be provided.

In the foot switch 108 shown in Fig. 7, operating buttons 136a and 136b and a stop button 138 for operations in the automatic mode are provided. The operating buttons 136a and 136b have the same functions as those of the operating buttons 132a and 132b of the remote controller 104. The foot switch 108 is placed below the assist table 2, as shown in Fig. 1. In the automatic mode, therefore, the operator can make progress in the process of operating the apparatus by using his or her foot. The arrangement may be such that in the manual mode the foot switch 108 is operated by being depressed to adjust the rate of flow of air supplied to or inhaled from the first balloon 42 or the second balloon 72.

Fig. 8 is a block diagram showing the internal configuration of the balloon control apparatus 100. As shown in Fig. 8, pump units 140 and 142 are provided in the main unit 102. The pump unit 140 communicates with the first balloon 42 and the pressure sensor 146 through the electromagnetic valve unit 144, while the pump unit 142 communicates with the second balloon 72 and the pressure sensor 150 through the electromagnetic valve unit 148. Each of the pump units 140 and 142 has a pressurizing pump and a depressurizing pump (not shown). A switching operation is performed in each electromagnetic valve units 144 and 148 to make one of the pressurizing pump and the depressurizing pump communicate with the balloon. The pump units 140 and 142 and the electromagnetic valve units 144 and 148 are connected to a central processing unit (CPU) 152. An operation to cause the first balloon 42 to expand or contract and an operation to cause the second balloon 72 to expand or contract are controlled by the CPU 152. That is, an operation to supply air from the pump unit 140 to cause the first balloon 42 to expand, an operation to inhale air by the pump unit 140 to cause the first balloon 42 to contract, an operation to supply air from the pump unit 142 to cause the second balloon 72 to expand and an operation to inhale air by the pump unit 142 to cause the second balloon 72 to contract are performed. During each operation, the pump unit 140 or 142 and the electromagnetic valve unit 144 or 148 are controlled according to the measured value from the pressure sensor 146 or 150. In this way, the internal pressure in the first balloon 42 or the second balloon 72 can be controlled at a predetermined level. If a fault (e.g., a burst of the first balloon 42 or the second balloon 72) occurs, the fault can be detected through the measured value from the pressure sensor 146 or 150 and supply or inhalation of air can be stopped.

The strain gauges 82 and 86 are connected to the CPU 152. When the measure value from the stain gauge 82 or 86 exceeds the threshold value, the electromagnetic valve unit 144 or 148 is controlled. Air is allowed to leak from the first balloon 42 or the second balloon 72, thereby causing the first balloon 42 or the second balloon 72 to contract.

A control unit 154 for controlling the holding device 200 is also connected to the CPU 152. Through this control unit 154, drive of the motors 220 and 240 of the holding device 200 is controlled. Further, the CPU 152 is connected to the computation device 228 of the holding device 200 and makes a determination as to whether or not insertion of the insertion portion 12 should be continued (whether or not drive of the motor 220 and 240 should be performed) on the basis of the entire amount of insertion obtained by the computation device 228.

A program input unit 156 is connected to the CPU 152 to enable a program to be input through an external input device 158 such as a keyboard. The input program is stored in a program memory 160. The program is a program for an operating process performed in the automatic mode. When this program is input, set values necessary in operations to be performed (including the set value of the pressure sensor 146 at the time of expansion/contraction of the first balloon 42, the set value of the pressure sensor 150 at the time of expansion/contraction of the second balloon 72, the amount of sliding of the insertion portion holder 210 at the time of insertion/drawing-out of the insertion portion 12, the amount of sliding of the assist implement holder 230 at the time of insertion/drawing-out of the insertion assist implement 70, the threshold values of the strain gauges 82 and 86 and a set value of the entire amount of insertion obtained by the computation device 228) are simultaneously input. The external input device 158 is also used when the program is modified in the learning mode described below.

An operating signal input interface (I/F) 162 is connected to the CPU 152. Through the operation signal input I/F 162, operating signals from the foot switch 108 and operating portions of the remote controller 104 (i.e., the mode change switch 128, the operating buttons 130a to 130j, operating buttons 132a and 132b, and the stop button 134) are input to the CPU 152. The CPU 152 outputs control signals to the above-described pump units 140 and 142, electromagnetic valve units 144 and 148 and control unit 154 according to the operating signals.

A program display unit 164 and a state display unit 166 are also connected to the CPU 152. The program display unit 164 and the state display unit 166 are connected to the balloon monitor 106 and the state display portion 126 of the remote controller 104 via the display switching unit 168. Therefore, not only the states of expansion/contraction of the first balloon 42 and the second balloon 72 but also the program can be displayed on the balloon monitor 106 and the state display portion 126.

The processor 30 is connected to the state display unit 166 to enable superimposition of images of the states of expansion/contraction of the first balloon 42 and the second balloon 72 on an image obtained by the endoscope 10.

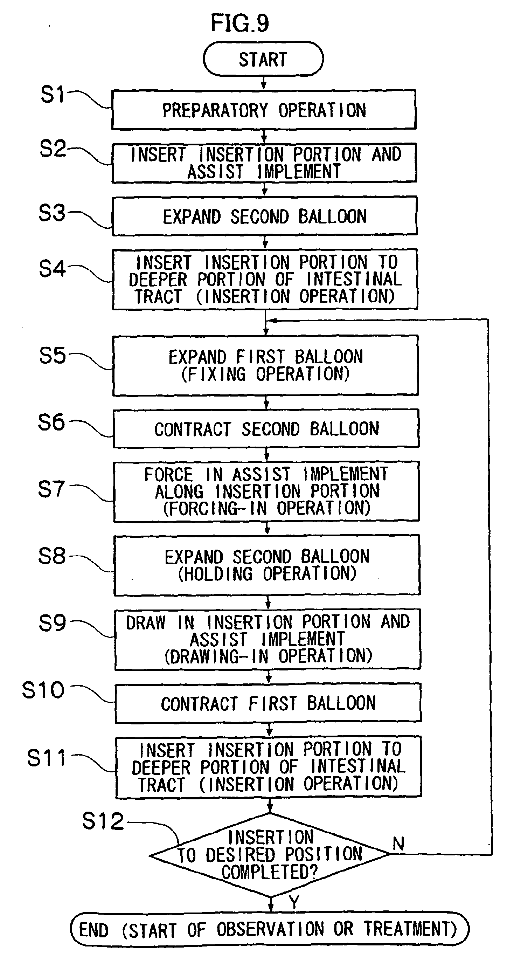

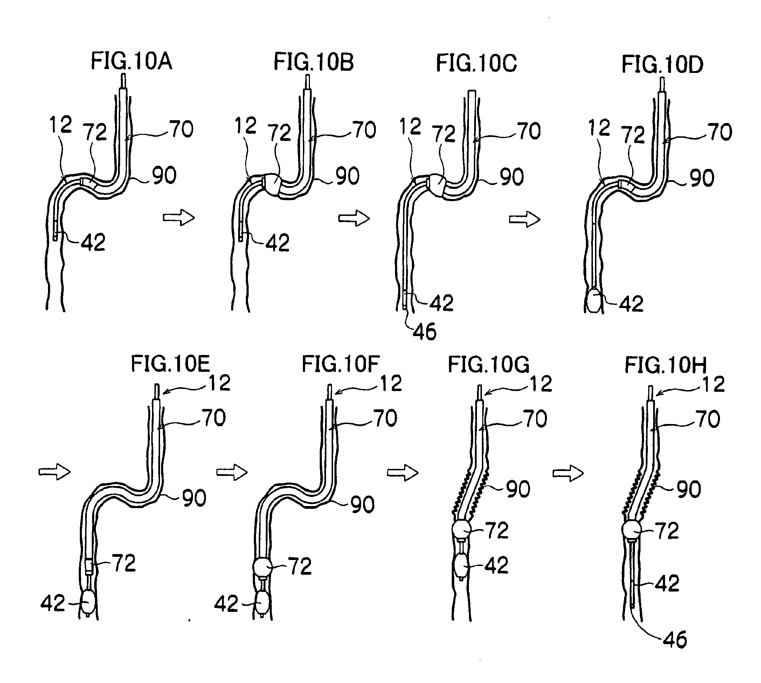

The method of operating the endoscope apparatus arranged as described above will now be described with reference to Figs. 9 and 10A to 10H. Fig. 9 is a flowchart showing a procedure by which the endoscope apparatus is operated. Figs. 10A to 10H are diagrams explaining a procedure by which the endoscope apparatus is operated.

Preparatory operations are first performed as a preparation to insertion operations to fit the insertion assist implement 70 around the insertion portion 12, fix the at-hand operating portion 14 of the endoscope 10 on the endoscope holder 210 and fix the insertion assist implement 70 on the assist implement holder 230 (step S1). At this time, the first balloon 42 and the second balloon 72 are kept in a shrunken state.

Operations described below are performed by the mode change switch 128 shown in Fig.7 selecting one of the automatic mode, the manual mode and the learning mode. Description will first be made of a case where the automatic mode is selected. When the automatic mode is selected, the operating button 132a of the remote controller 104 or the operating button 136a of the foot switch 108 (hereinafter referred to collectively as "progress button") is operated by being depressed to allow each operating step to proceed automatically.

For example, when the progress button is depressed after the above-mentioned preparatory operations, the motors 220 and 240 shown in Fig. 4 are driven to cause the endoscope holder 210 and the assist implement holder 230 to advance by the predetermined amounts toward the mouth 4 of the patient 1 and stop. The insertion portion 12 and the insertion assist implement 70 are inserted into a body cavity (step S2) until the fore end of the insertion assist implement 70 reaches a bend in an intestinal tract 90, as shown in Fig. 10A.

When the progress button is thereafter depressed, air is supplied to the second balloon 72 by controlling the pump unit 142 and the electromagnetic valve unit 148 shown in Fig. 8. Supply of air to the second balloon 72 is continued until the measured value from the pressure sensor 150 falls into a predetermined range. The second balloon 72 is thereby expanded (step S3). The insertion assist implement 70 is fixed in the intestinal tract 90 by the expanded second balloon 72, as shown in Fig. 10B.

When the progress button is operated by being depressed in this state, the motor 220 shown in Fig. 4 is driven to cause the endoscope holder 210 to advance toward the mouth 4 of the patient 1. The endoscope holder 210 is stopped when a predetermined amount of advancement is reached. At this time, the operator operates the angle knobs 38 of the at-hand operating portion 14 to bend the bending portion 48 (see Fig. 2) of the insertion portion 12 by following the bent shape of the intestinal tract 90. The insertion portion 12 is thereby inserted in the intestinal tract 90 (step S4) until the fore end of the insertion portion 12 is inserted to a deeper portion of the intestinal tract 90, as shown in Fig. 10C. During the operation to insert the insertion portion 12, the amount of insertion of the insertion portion 12 is measured by the insertion amount measurement device constituted by the roller 224, the gear 226 and the sensor 227 and the measured value of the amount of insertion is integrated by the computation device 228 to obtain the entire amount of insertion.

When the progress button is subsequently operated by being depressed, air is supplied to the first balloon 42 by controlling the pump unit 140 and the electromagnetic valve unit 144 shown in Fig. 8. Supply of air to the first balloon 42 is continued until the measured value from the pressure sensor 146 falls into a predetermined range. The first balloon 42 is thereby expanded (step S5). The insertion portion 12 is fixed in the intestinal tract 90 by the expanded first balloon 42, as shown in Fig. 10D.

When the progress button is depressed in this state, air is inhaled from the second balloon 72 by controlling the pump unit 142 and the electromagnetic valve unit 148 shown in Fig. 8. Inhalation of air from the second balloon 72 is continued until the measured value from the pressure sensor 150 falls into a predetermined range. The second balloon 72 is thereby shrunk (step S6) to adhere to the surface of the insertion assist implement 70, as shown in Fig. 10D.

When the progress button is subsequently depressed, the motor 240 shown in Fig. 4 is driven to move the assist implement holder 230 toward the mouth 4 of the patient 1. Thus, the insertion assist implement 70 is inserted into the patient 1 while being linearly guided. The assist implement holder 230 is stopped when the predetermined amount of movement is reached. The insertion assist implement 70 is thereby inserted by the predetermined amount (step S7) to place the second balloon 72 after the first balloon 42 in the vicinity of the same, as shown in Fig. 10E. At this time, since the amount of insertion of the insertion assist implement 70 can be controlled by controlling the drive of the motor 24, contact of the fore end of the insertion assist implement 70 with the first balloon 42 of the insertion portion 12 can be prevented and, hence, damage on the first balloon 42 can be prevented.

When the progress button is depressed in the state where the insertion assist implement 70 is inserted, air is supplied to the second balloon 72 by controlling the pump unit 142 and the electromagnetic valve unit 148 shown in Fig. 8. Supply of air to the second balloon 72 is continued until the measured value from the pressure sensor 150 falls into the predetermined range. The second balloon 72 is thereby expanded (step S8) to fix insertion assist implement 70 in the intestinal tract 90, as shown in Fig. 10F. That is, the intestinal tract 90 is held by the second balloon 72.

When the progress button is operated in this state, the motors 220 and 240 shown in Fig. 4 are driven to simultaneously move the endoscope holder 210 and the assist implement holder 230 by the predetermined amount away from the mouth 4 of the patient 1. The insertion portion 12 and the insertion assist implement 70 are thereby drawn out simultaneously through the mouth 4 of the patient 1 to draw in the intestinal tract 90 (step S9). As a result, the intestinal tract 90 contracts and the unnecessary bend or warp in the insertion assist implement 70 is removed, as shown in Fig. 10G. If the measured value from the strain gauge 82 or 86 (see Fig. 2) exceeds the threshold value during drawing in, the balloon control apparatus 100 allows air to leak from the first balloon 42 or the second balloon 72 to cause the first balloon 42 or the second balloon 72 to contract. The drawing-out force is thereby reduced to prevent imposition of a large load on the intestinal tract 90.

When the progress button is depressed in the state where the insertion portion 12 and the insertion assist implement 70 are drawn in, air is inhaled from the first balloon 42 by controlling the pump unit 140 and the electromagnetic valve unit 144 shown in Fig. 8. Inhalation of air from the first balloon 42 is continued until the measured value from the pressure sensor 146 falls into a predetermined range. The first balloon 42 is thereby shrunk (step S10) to adhere to the surface of the insertion portion 12, as shown in Fig. 10H.

When the progress button is subsequently depressed, the motor 220 shown in Fig. 4 is driven to cause the endoscope holder 210 to move by the predetermined amount toward the mouth 4 of the patient 1 and stop. The insertion portion 12 is thereby inserted to a deeper portion of the intestinal tract 90 (step S11). At this time, since the unnecessary bend or warp of the insertion assist implement 70 has been removed, the insertion portion 12 can be smoothly inserted.

When the insertion portion 12 is inserted to a deeper portion of the intestinal tract 90, the balloon control apparatus 100 determines whether or not the fore end of the insertion portion 12 has reached a predetermined position, that is, the entire amount of insertion obtained by the computation device 228 shown in Fig. 6 has reached a set value (step S12). If the set value has not been reached, the above-described operations (steps S5 to S11) are repeated. That is, the fixing operation shown in Fig. 10D is performed, the forcing-in operation shown in Fig. 10E is thereafter performed, and the holding operation shown in Fig. 10F, the drawing-in operation shown in Fig. 10g and the insertion operation shown in Fig. 10H are further repeated, thereby inserting the insertion portion 12 to a further deeper portion of the intestinal tract 90. After the entire amount of insertion has reached the desired value, observation or treatment through the endoscope 10 is performed.

Thus, in this embodiment, the above-described complicated operations (steps S2 to S12) can be automatically performed if only the operation to depress the progress button is performed. The operator can easily perform the operation with no need to consider the operating procedure.

In the above-described automatic mode, a return to the preceding operation can be made by operating the operating button 132b of the remote controller 104 or the operating button 136b, and each operation can be stopped by depressing the stop button 134 or 138.

Description will next be made of operations in the manual mode. In the manual mode, the operator selects and depresses one of the operating buttons 130a to 130j shown in Fig. 7 to advance each of operations described below. That is, after the completion of the preparatory operations shown in Fig. 9 (step S1), the operating button 130c is depressed to insert the insertion portion 12 and the insertion assist implement 70 (step S2). Subsequently, the operating button 130i is depressed to expand the second balloon 72 (step S3) and the operating button 130a is depressed to insert the insertion portion 12 to a deeper portion of the intestinal tract (step S4). The operating button 130g is thereafter depressed to expand the first balloon 42 (step S5). Subsequently, the operating button 130j is depressed to contract the second balloon 72 (step S6) and the operating button 130b is thereafter depressed to force in the insertion assist implement 70 along the insertion portion 12 (step S7). Subsequently, the operating button 130i is depressed to expand the second balloon 72 (step S8) and the operating button 130f is depressed to draw in the insertion portion 12 and the insertion assist implement 70 (step S9). Subsequently, the operating button 130h is depressed to contract the first balloon 42 (step S10) and the operating button 130a is depressed to insert the insertion portion 12 to a deeper portion of the intestinal tract 90 (step S11). Thus, in the manual mode, one of the operating buttons 130a to 130j is selectively depressed to perform each operation. Therefore, the endoscope apparatus can be operated by an operating procedure not provided as a program in a memory.

Preferably, lamps such as light emitting diodes (LEDs) are provided in the operating buttons 130a to 130j for the operations in the manual mode; the operating buttons 130a to 130j are formed so that lighting of the lamps can be recognized; and each of the operating buttons 130a to 130j is lighted when the corresponding operation is performed, thereby enabling the operator to always grasp which operation is being performed. Also, one of the operating buttons 130a to 130j corresponding to the next operation may be lighted in a colour different from a color in which the operating button corresponding to the preceding operation is lighted for guidance to the next operation. Also in the automatic mode, the manual-mode operating buttons 130a to 130j may be lighted for understanding of the operating state.

In this embodiment, the learning mode can also be selected. In the learning mode, each operation can be performed by selecting and depressing the corresponding one of the operating buttons 130a to 130j shown in Fig. 7, as is each operation in the manual mode. After the completion of all the operations, the sequence of operations is displayed on the display (the balloon monitor 106, the state display portion 126 of the remote controller 104, or the like). The operator checks the sequence of operations which has been displayed. If some portion of the sequence should be corrected, the operator corrects it through the external input means 158, e.g., a keyboard (see Fig. 8) and newly stores the corrected sequence as the program for operations in the automatic mode. In this way, an operating procedure executed in the learning mode can be executed for operations in the automatic mode the next time the apparatus is used. Thus, in this embodiment, a change in the program or an addition to the program can be made when the learning mode is selected.

In the endoscope apparatus in this embodiment, as described above, a program representing an operating procedure by which operations to expand or contract the first and second balloons 42 and 72 and operations to insert or drawing out the insertion portion 12 and the insertion assist implement 70 are performed is stored in the balloon control apparatus 100. When the automatic mode is selected, the progress button (operating button 132a or 136s) is operated to enable each operation to advance in accordance with the stored operating procedure. Therefore, the operator may operate only the progress button and can concentrate on operations relating to observation and treatment through the endoscope 10.

According to this embodiment, operations can be performed by an operating procedure different from the stored program when the manual mode is selected.

Further, according to this embodiment, an operating procedure by which operations are manually performed when the learning mode is selected can be used in the automatic mode.

While this embodiment has been described with respect to the operating procedure shown in Fig. 9, the operating procedure shown in Fig. 9 is not exclusively used. Programs for operating procedures in various patterns may be stored and executed. For example, in the operating procedure shown in Fig. 9, the operation to insert the insertion portion 12 (step S4, step S11) and the operation to insert the insertion assist implement 70 (step S7) may be manually performed and a program for the operating procedure formed by removing these manual operations may be stored and executed.

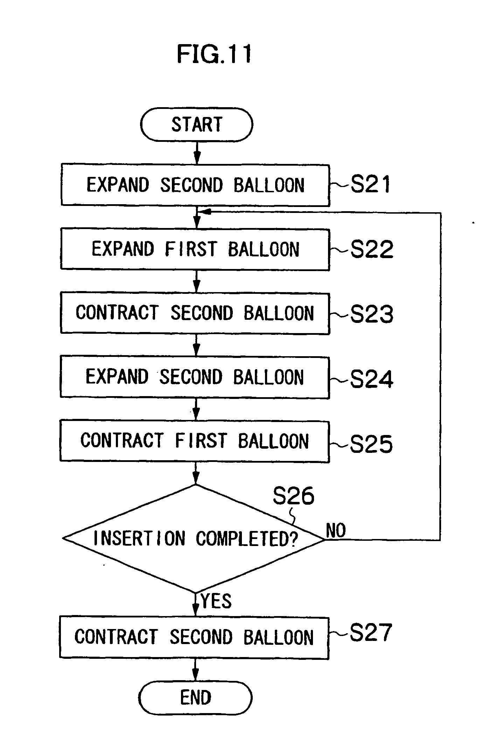

Also, a program representing an operating procedure by which only operations to expand and contract the first and second balloons 42 and 72 are performed may be stored and executed as shown in Fig. 11. Description will be made of the operations in this case. The operator first fits the insertion assist implement 70 around the insertion portion 12 of the endoscope 10, manually inserts the insertion portion 12 and the insertion assist implement 70 into a body cavity of the patient 1, and depresses the progress button when the fore end of the insertion assist implement 70 reaches a bend in the intestinal tract 90. The second balloon 72 is thereby expanded to fix the insertion assist implement 70 in the intestinal tract 90 (step S21). Subsequently, the operator manually inserts the insertion portion 12 to a deeper portion of the intestinal tract 90 and depresses the progress button. The first balloon 42 is thereby expanded to fix the insertion portion 12 in the intestinal tract 90 (step S22). When the operator further performs the progress button depressing operation, the second balloon 72 contracts (step S23). In this state, the operator manually forces in the insertion assist implement 70 along the insertion portion 12 and depresses the progress button when the second balloon 72 is brought close to the first balloon 42. The second balloon 72 is thereby expanded to fix the insertion assist implement 70 in the intestinal tract 90 (step S24). In this state, the operator manually draws in the insertion portion 12 and the insertion assist implement 70. The operator thereafter depresses the progress button to cause the first balloon 42 to contract (step S25). Then the operator manually inserts the insertion portion 12 to a deeper portion of the intestinal tract 90. If the insertion portion 12 should be caused to advance to a further deeper portion of the intestinal tract 90 (step S26), the operator depresses the progress button to again expand the first balloon 42, and the above-described operations (steps S22 to S25) are repeated. The insertion portion 12 can be thereby inserted into a further deeper portion of the intestinal tract 90. After the completion of the entire process, the second balloon 72 is caused to contract (step S27). Also in such a case where only the operations to expand and contract the first and second balloons 42 and 72 are stored and executed, the operator can concentrate on the operation to insert and draw out the insertion portion 12 of the endoscope 10 and the operation to insert and draw out the insertion assist implement 70, thus reducing the burden.

Similarly, in a case where only the second balloon 72 of the insertion assist implement 70 is provided (that is, the first balloon 42 is not attached to the insertion portion 12), a program representing an operating procedure relating to the operations to expand and contract the second balloon 72 may be stored and executed.

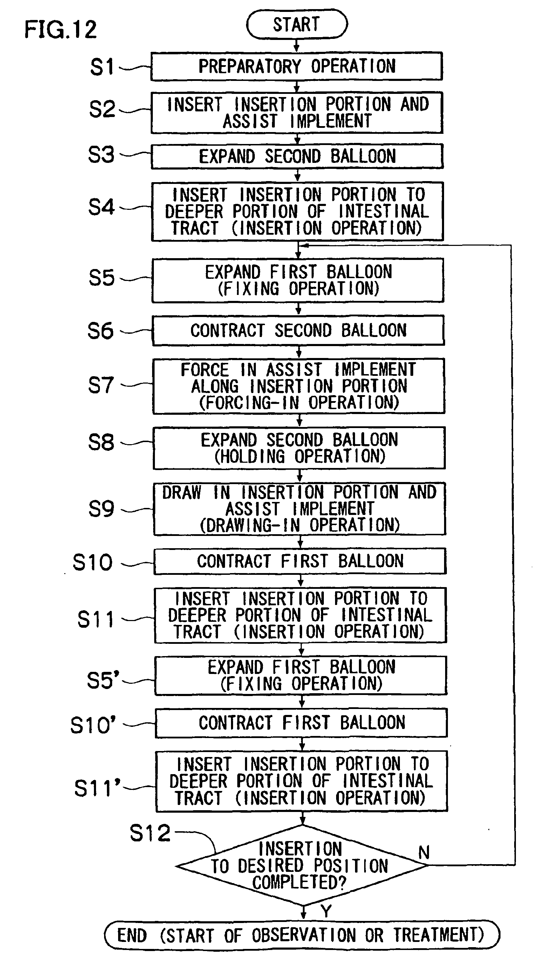

Operating procedures shown in the flowcharts of Figs. 12 to 18 may also be stored and executed. In the flowcharts of Figs. 12 to 18, the same operations as those shown in Fig. 9 are indicated by the same reference numerals. The description for the same operations will not be repeated.

The flowchart shown in Fig. 12 represents an operating procedure in which the operation to insert the insertion portion 12 is continuously performed two times. In this operating procedure, the operations before insertion of the insertion portion 12 and the operation to insert the insertion portion 12 (i.e., steps S1 to S11) are performed in the same manner as those in the operating procedure shown Fig. 9. After the insertion operation in step S11, the first balloon 42 is expanded to fix the insertion portion 12 in the intestinal tract 90 (step S5'), as in step S5. Subsequently, the first balloon 42 is shrunk (step S10'), as in step S10, and the insertion portion 12 is inserted (step S11'), as in step S11. Thereafter, determination is made as to whether or not the fore end of the insertion portion 12 has reached the desired position (step S12) and the same processing as that in the flowchart of Fig. 9 is performed. This operating procedure is used, for example, in a case where observation is performed during insertion of the insertion portion 12. While an example of an operating procedure in which the insertion portion 12 is continuously inserted two times has been described, the insertion portion 12 may be continuously inserted three or more times by repeating steps S5', S10', and S11'.

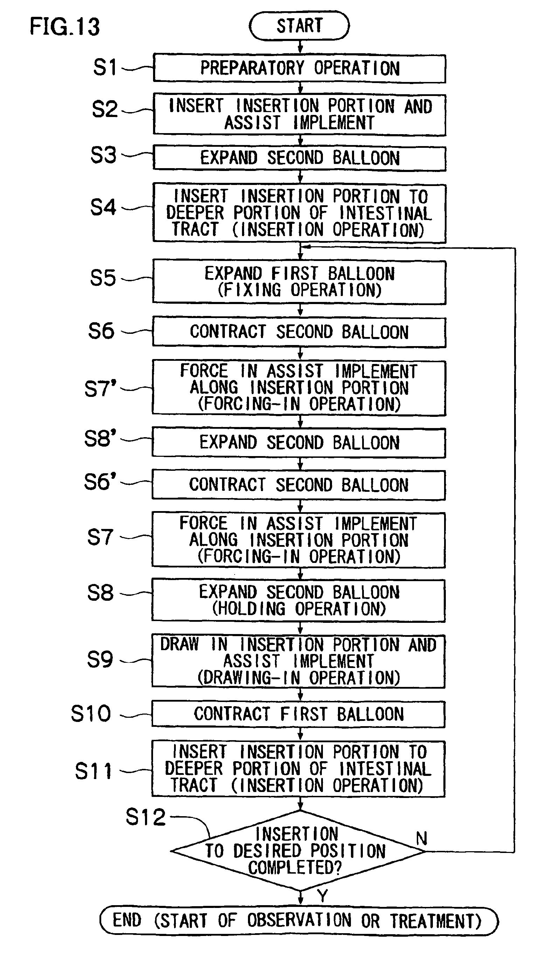

The flowchart shown in Fig. 13 represents an operating procedure in which the insertion assist implement 70 is forced in two stages. In this operating procedure, the operations before contraction of the second balloon 72 and the operation to contract the second balloon 72 (i.e., steps S1 to S6) are performed in the same manner as those in the operating procedure shown in Fig. 9. After the operation to contract the second balloon 72 in step S6, the insertion assist implement 70 is forced in along the insertion portion 12 (step S7'), as in step S7, the second balloon 72 is expanded in an intermediate step to temporarily fix the insertion assist implement 70 in the intestinal tract 90 (step S8'), and the second balloon 72 is thereafter shrunk (step S6'). The insertion assist implement 70 is again forced in along the insertion portion 12, and the second balloon 72 is expanded when the fore end of the insertion assist implement 70 is placed in the vicinity of the first balloon 42 (steps S7, S8). The other processing steps are performed in the same manner as those in the flowchart of Fig. 9. This operating procedure is performed, for example, in a case where the insertion portion 12 is bent so that the insertion assist implement 70 cannot be easily forced in. While an example of an operating procedure in which the insertion assist implement 70 is continuously forced in two times has been described, the insertion assist implement 70 may be continuously inserted three or more times.

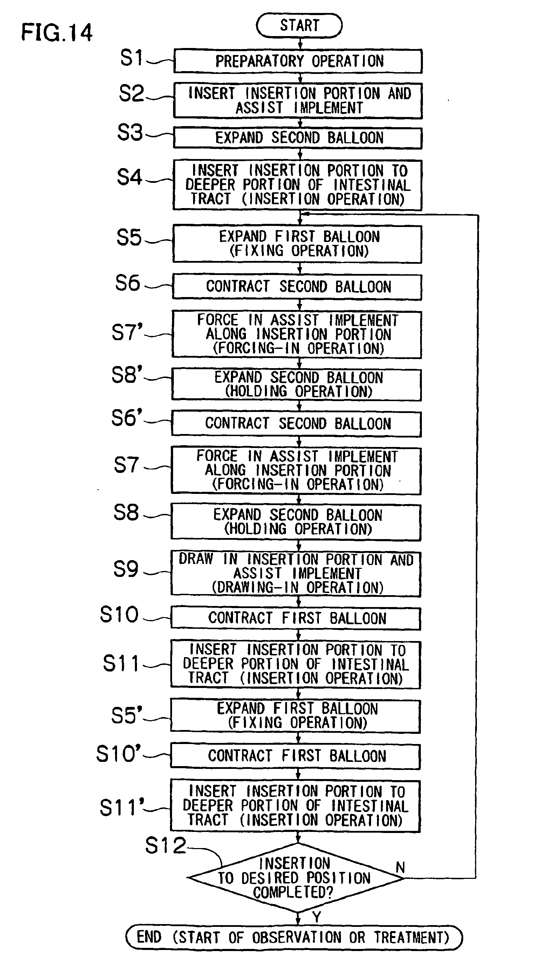

The flowchart shown in Fig. 14 represents an operating procedure in which the insertion portion 12 is continuously inserted two times, as in the procedure shown in the flowchart of Fig. 12, and in which the insertion assist implement 70 is continuously forced in two times, as in the procedure shown in the flowchart of Fig. 13. That is, the flowchart of Fig. 14 is formed by incorporating steps S7', S8', and S6' of the flowchart of Fig. 13 and steps S5', S10', and S11' of the flowchart of Fig. 12 in the flowchart of Fig. 9.

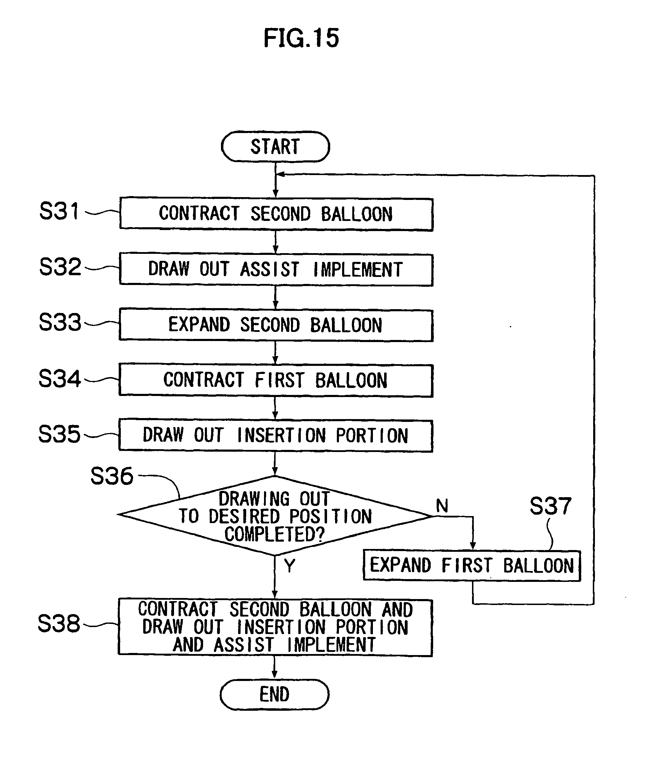

Flowcharts shown in Figs. 15 to 18 show examples of operating procedures for drawing out the insertion portion 12 and the insertion assist implement 70 from the intestinal tract 90. Fig. 15 shows a basic operating procedure. The process shown in these flowcharts is started in a state where the insertion portion 12 and the insertion assist implement 70 are inserted to a deeper portion of the intestinal tract 90, and where the first and second balloons 42 and 72 are in the expanded state.

As shown in Fig. 15, the first balloon 72 is first shrunk (step S31) for drawing out. The insertion assist implement 70 is drawn out by the desired amount (step S32) and the second balloon 72 is thereafter expanded to fix the insertion assist implement 70 in the intestinal tract 90 (step S33). Subsequently, the first balloon 42 is shrunk (step S34) and the insertion portion 12 is drawn out (step S35). Determination is made as to whether or not the fore end of the insertion portion 12 has been drawn out to a desired position (e.g., the duodenum) (step S36). If the fore end of the insertion portion 12 has not been drawn out to the desired position, the first balloon 42 is again expanded (step S37) and steps S31 to S35 are repeated. After the fore end of the insertion portion 12 has been drawn out to the desired position, the second balloon 72 is shrunk and the insertion portion 12 and the insertion assist implement 70 are simultaneously drawn out from the body cavity (step S38), thus completing the process.

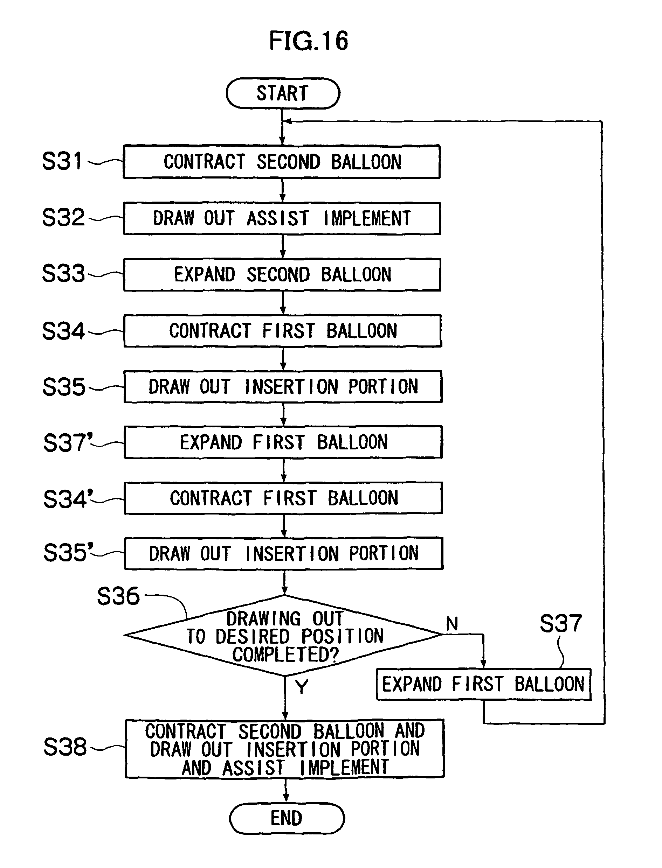

The flowchart shown in Fig. 16 shows an operating procedure in which the insertion portion 12 is drawn out in two stages. In this operating procedure, the operations before drawing out of the insertion portion 12 and the operation to draw out the insertion portion 12 (i.e., steps S31 to S35) are performed in the same manner as those in the operating procedure shown in Fig. 15. After the insertion portion 12 has been drawn out in step S35, the first balloon 42 is expanded to fix the insertion portion 12 in the intestinal tract 90 (step S37'), as in step S37. Subsequently, the first balloon 42 is shrunk (step S34'), as in step S34, and the insertion portion 12 is drawn out (step S35'), as in step S35. The subsequent processing steps are performed in the same manner as those shown in the flowchart of Fig. 15. This processing procedure is used, for example, in a case where observation is performed by fixing the insertion portion 12 in the process of drawing out. While an example of an operating procedure in which the insertion portion 12 is continuously drawn out two times has been described, the insertion portion 12 may be continuously drawn out three or more times.

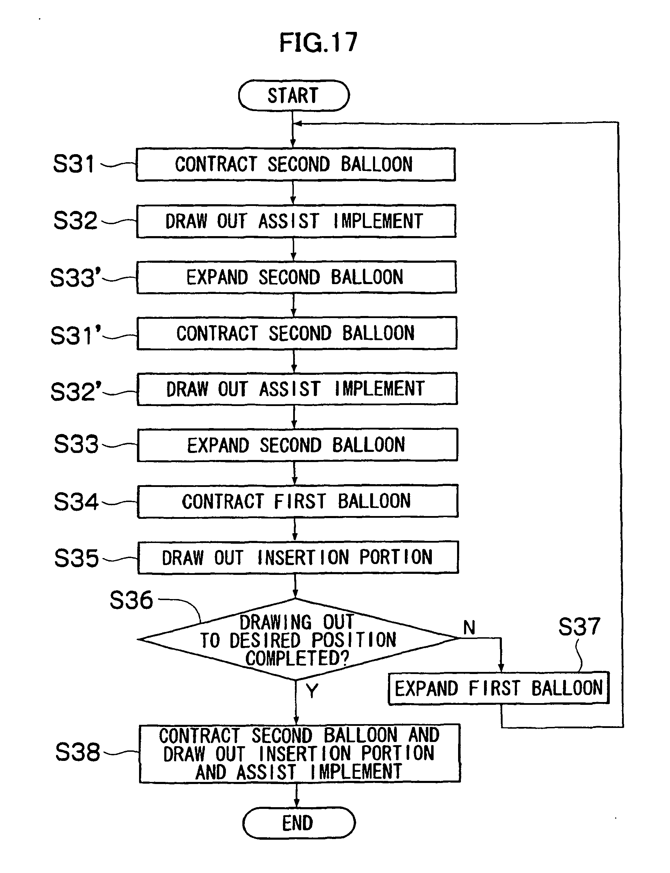

The flowchart shown in Fig. 17 represents an operating procedure in which the insertion assist implement 70 is drawn out in two stages. In this operating procedure, the operation to contract the second balloon 72 and the operation before drawing out the insertion assist implement 70 and the operation to draw out the insertion assist implement 70 (i.e., steps S31 and S32) are performed in the same manner as those in the operating procedure shown in Fig. 15. After the insertion assist implement 70 has been drawn out in step S32, the second balloon 72 is expanded to temporarily fix the insertion assist implement 70 in the intestinal tract 90 (step S33'), as in step S33, and the second balloon 72 is shrunk and the insertion assist implement 70 is drawn out (steps S31' and S32'), as in steps S31 and S32. Subsequently, the second balloon 72 is expanded to fix the insertion assist implement 70 in the intestinal tract 90. The subsequent processing steps are performed in the same manner as those in the flowchart of Fig. 15. This operating procedure is performed, for example, in a case where the insertion assist implement 70 is gradually drawn out without imposing a burden on the intestinal tract 90. While an example of an operating procedure in which the insertion assist implement 70 is continuously drawn out two times has been described, the insertion assist implement 70 may be continuously drawn out three or more times.

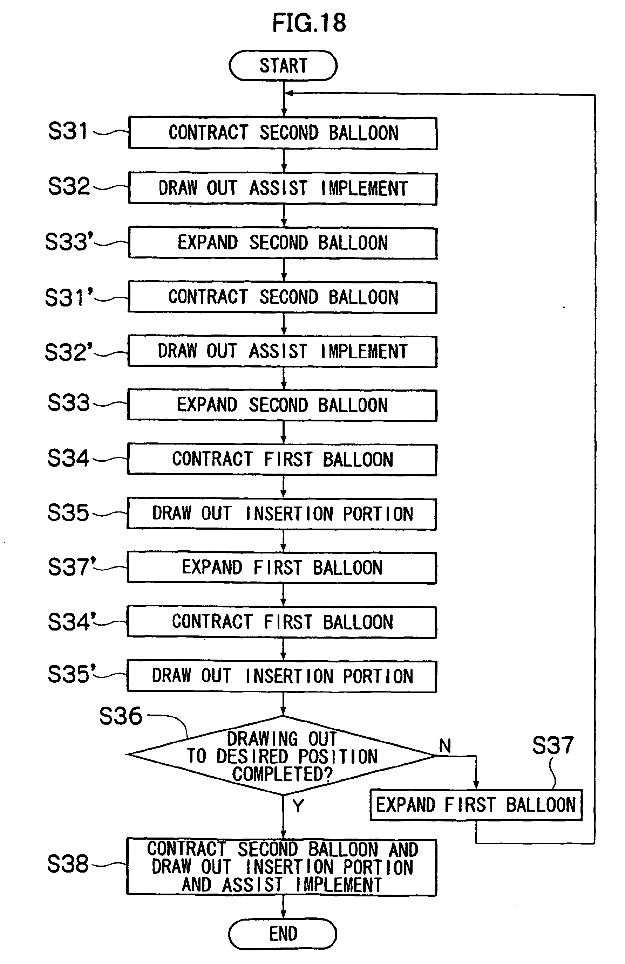

The flowchart shown in Fig. 18 represents an operating procedure in which the insertion portion 12 is continuously drawn out two times, as in the procedure shown in the flowchart of Fig. 16, and in which the insertion assist implement 70 is continuously drawn out two times, as in the procedure shown in the flowchart of Fig. 17. That is, the flowchart of Fig. 18 is formed by incorporating steps S33', S31', and S32' of the flowchart of Fig. 17 and steps S37', S34', and S35' of the flowchart of Fig. 16 in the flowchart of Fig. 15.

If there are a plurality of operating procedures in different patterns such as those described above, it is preferable to input all the operating procedures and to enable selection of a desired one of the input operating procedures. In such a case, a selecting switch may be provided in the remote controller 104 and the main unit 102. Also in a case where the set values vary from patient to patient even when one operating procedure pattern is used, the arrangement may be such that the various set values can be individually stored and selected.

While in the above-described embodiment one of the automatic mode, the manual mode and the learning mode is selected by using the mode change switch 128, the arrangement may be such that selection from the modes is automatically performed when one of the operating buttons 130a to 130j and the operating buttons 132a and 132b is operated. For example, the manual mode may be selected when one of the operating buttons 130a to 130j is operated during execution of the automatic mode. Conversely, when the operating button 132a or 132d is operated during execution of the manual mode, the present stage of operation may be automatically identified and a transition to the automatic mode may be made according to the identified stage of operation.

While in the above-described embodiment the operating buttons 132a and 132b are used as the execution direction device, the positions of such operating buttons for the execution direction device are not limited to those in the remote controller 104. The operating buttons may be provided in the at-hand operating portion 14 of the endoscope 10 or the base end portion 74 of the insertion assist implement 70. The execution direction device is not limited to the operating buttons. A direction to execute the desired operation may be given by means of sound such as voice.

While in the above-described embodiment the endoscope 10 and the insertion assist implement 70 are linearly guided by the holding device 200, any device other than the linear guide may suffice if it is capable of movably supporting the endoscope 10 and the insertion assist implement 70. Description will be made of examples of the holding device 200.

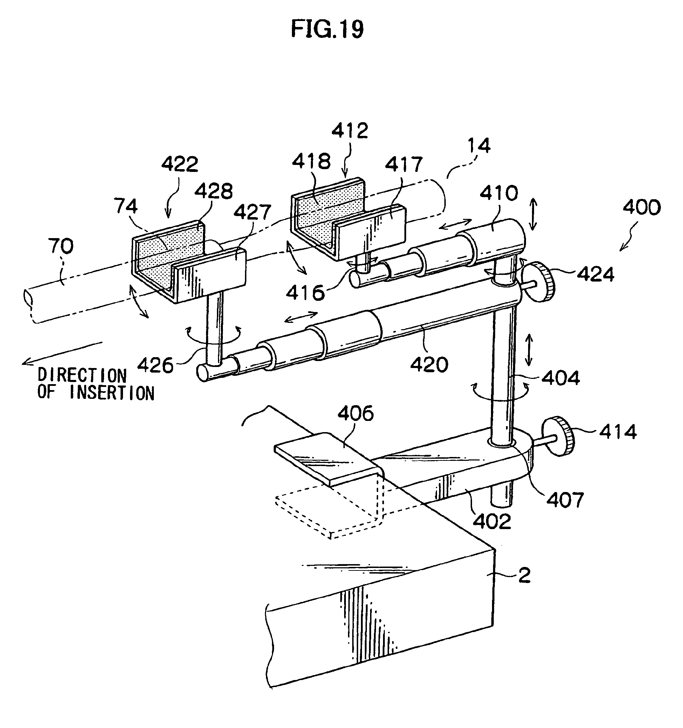

An example of the holding device in another embodiment of the present invention will be described. As shown in Fig. 19, a holding device 400 is constituted by a fixed base portion 402, a supporting post 404, arms 410 and 420 provided on the supporting post 404 and extending in a direction perpendicular to the axial direction of the supporting post 404, and holders 412 and 422 provided on the arms 410 and 420 in the vicinity of the extreme ends of the arms 410 and 420. The fixed base portion 402 has a clamp 406 by which the examination table 2 is clamped to fix the fixed base portion 402 on the examination table 2. Any device other than the clamp 406, e.g., a device using magnetism or screws may be used as a device for fixing the fixed base portion 402.

The supporting post 404 is vertically disposed, passed through a through hole 407 formed in the fixed base portion 402, and fixed on the fixed base portion 402 by tightening a fixing screw 414. When the fixing screw 414 is loosened, the supporting post 404 can be moved in the vertical direction.

The arm 410 horizontally disposed is fixed on the upper end of the supporting post 404. The position of the arm 410 in the height direction can be adjusted by vertically moving the supporting post 404 relative to the fixed base portion 402.

The arm 420 horizontally disposed is mounted on the supporting post 404 so as to be vertically movable relative to the supporting post 404. The arm 420 can be fixed in a desired position in the height direction by fastening an adjusting screw 424.

Each of the arms 410 and 420 is constituted of a plurality of tubular members combined so as to form a telescopic tube capable of extending horizontally. Connecting members 416 and 426 in the form of rods are vertically mounted on end portions of the arms 410 and 420. Each of the connecting members 416 and 426 is supported so as to be rotatable about a vertical axis. Holders 412 and 422 are tiltably mounted on the connecting members 416 and 426. Suitable frictional forces act on the extending/contracting operations of the arms 410 and 420, the rotating operations of the connecting members 416 and 426 and the tilting operations of the holders 412 and 422 to enable each of the arms 410 and 420, connecting members 416 and 426 or holders 412 and 422 to be fixed in a desired position.

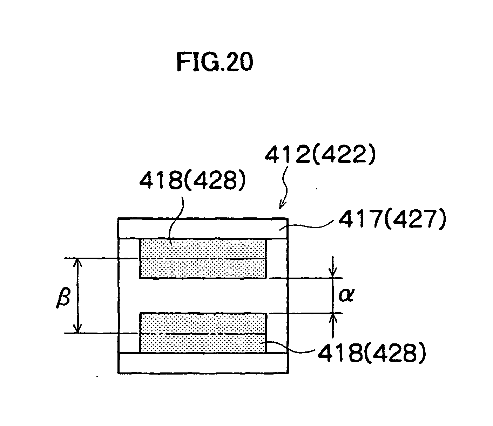

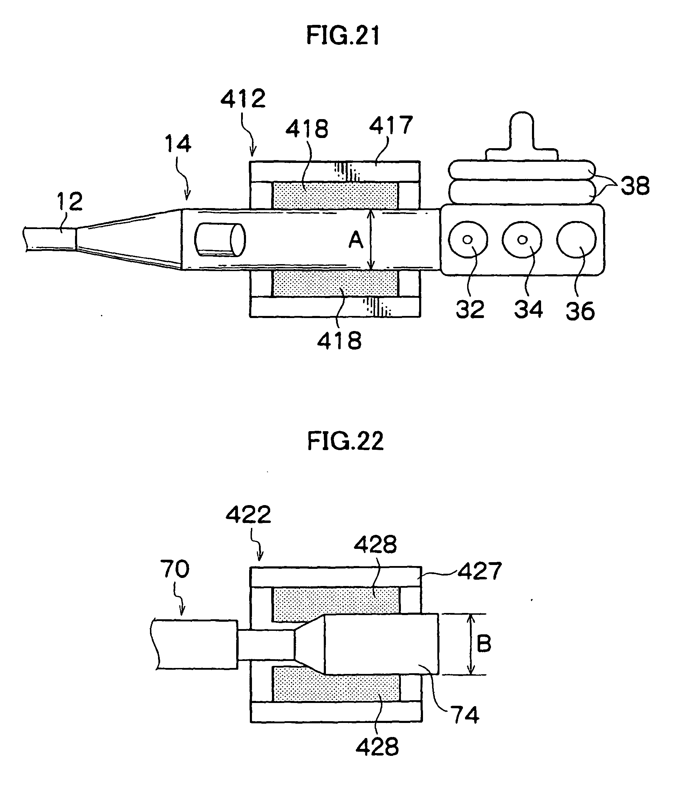

The holders 412 and 422 are respectively constituted of metallic supporting members 417 and 427 each formed so as to be generally U-shaped and elastic members 418 and 428 made of rubber, sponge or the like and placed inside the metallic supporting members 417 and 427. A setting of the elastic members 418 and 428 is made so as to satisfy a condition described below in order to ensure that the elastic members 418 and 428 can hold the at-hand operating portion 14 of the endoscope 10 and the base end portion 74 of the insertion assist implement 70 by their resilience. That is, if the spacing between each of the pairs of opposite portions of the elastic members 418 and 428 in a natural condition is α (see Fig. 20); the spacing when the amount of deformation of the elastic member is at the maximum is β; the width of the at-hand operating portion 14 is A (see Fig. 21); and width of the base end portion 74 of the insertion assist implement 70 is B (see Fig. 22), relationships expressed by α < A < β and α < B < β are satisfied. If such elastic members 418 and 428 are attached, the at-hand operating portion 14 and the base end portion 74 can be held by being only inserted in the holders 412 and 422 from above. Also, the at-hand operating portion 14 and the base end portion 74 can be detached from the holders 412 and 422 by being only drawn out upwardly. Further, since the holders 412 and 422 are formed so as to be generally U-shaped, the directions in which the at-hand operating portion 14 and the base end portion 74 are moved while being held in the holders 412 and 422 are limited to one direction (e.g., the direction of insertion) by the holders 412 and 422. The constructions of the holders 412 and 422 are not limited to that described above. Any other constructions in which the holders 412 and 422 have such shapes as to be capable of holding the endoscope 10 and the insertion assist implement 70 may be adopted.

The holding device 400 constructed as described above is used in such a manner that after the insertion portion 12 of the endoscope 10 and the insertion assist implement 70 have been inserted into the patient 1, the at-hand operating portion 14 of the endoscope 10 and the base end portion 74 of the insertion assist implement 70 are held by being respectively inserted in the holders 412 and 422. Thereafter, the insertion portion 12 or the insertion assist implement 70 is moved by extending or contracting the arm 410 or the arm 420. In this way, the insertion portion 12 and the insertion assist implement 70 are forced into a body cavity of the patient 1. Thus, in this embodiment, the need to operate the endoscope 10 and the insertion assist implement 70 while holding both the endoscope 10 and insertion assist implement 70 is eliminated to enable one operator to operate the endoscope 10 and the insertion assist implement 70.

In this embodiment, the angles of the at-hand operating portion 14 and the base end portion 74 held in the holders 412 and 422 in the direction of insertion can be freely changed by tilting the holders 412 and 422 relative to the connecting members 416 and 426 and rotating the connecting members 416 and 426. Also, the positions of the holders 412 and 422 can be freely adjusted by changing the height positions of the arms 410 and 420 and extending or contracting the arms 410 and 420. In this way, the position for insertion into the patient 1 can be freely adjusted. According to this embodiment, the insertion directions and the insertion positions of the endoscope 10 and the insertion assist implement 70 at the time of insertion into the patient 1 can be freely adjusted, and the endoscope 10 and the insertion assist implement 70 can therefore be set so as to be easily inserted into the patient, thus effectively reducing the burden on the patient 1.

In the above-described embodiment, the operations to extend or contract the arms 410 and 420, the operations to adjust the height positions of the arms 410 and 420, the operations to rotate the connecting members 416 and 426 and the operations to tilt the holders 412 and 422 are manually performed. The operations, however, may be automatically performed by using drive devices such as motors or cylinders. In such a case, the positions and attitudes of the holders 412 and 422 can be adjusted as desired by controlling the amounts of operation performed by the drive devices.

In the above-described embodiment, the endoscope 10 and the insertion assist implement 70 are moved while being held in the holders 412 and 422. This operating method, however, is not exclusively used. The endoscope 10 and the insertion assist implement 70 may be held by being fitted to the holders 412 and 422 only when necessary. Each of the endoscope 10 and the insertion assist implement 70 may be detached from the holder 412 or 422 when moved.

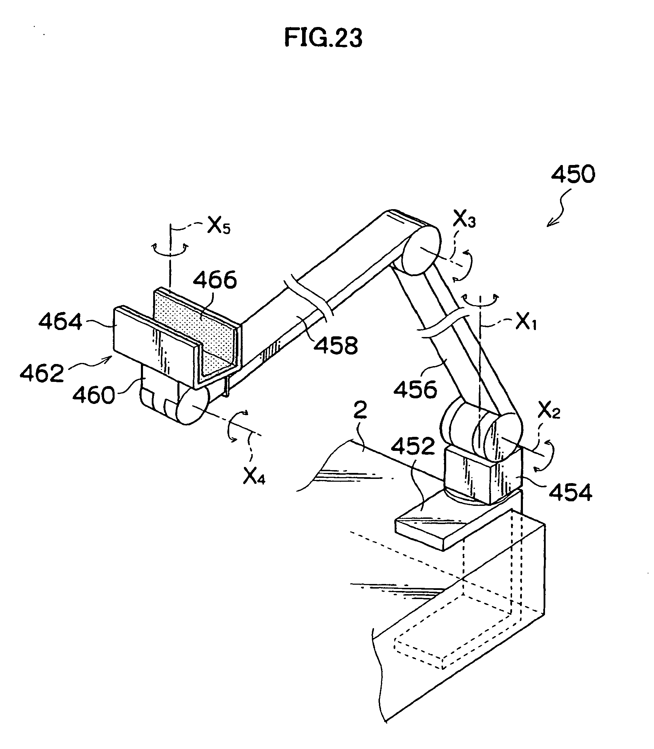

Fig. 23 shows an example of a holding device in which holders are supported by an arm mechanism different from that shown in Fig. 19. The holding device 450 shown in Fig. 23 is constituted of a fixed portion 452, a rotary base 454, arms 456 and 458, a connecting member 460 and a holder 462. The fixed portion 452 is fixed on the examination table 2 by clamping the examination table 2. The rotary base 454 is supported on the fixed portion 452 so as to be rotatable about a vertical axis X1. The lower end of the arm 456 is supported on the rotary base 454 so as to be rotatable about a horizontal axis X2. The arm 458 is supported on the upper end of the arm 456 so as to be rotatable about a horizontal axis X3. The connecting member 460 is supported on the distal end of the arm 458 so as to be rotatable about a horizontal axis X4. The holder 462 is supported on the connecting member 460 so as to be rotatable about a vertical axis X5.

The holder 462 is constituted of a metallic supporting member 464 formed so as to be generally C-shaped and an elastic member 466 placed inside the supporting member 464, as are the holders 412 and 422 shown in Fig. 19. The elastic member 466 is formed so as to satisfy α < A < β and α < B < β, as are the elastic members 418 and 428 shown in Figs. 20 to 22. The holder 462 is capable of holding either of the at-hand operating portion 14 and the base end portion 74.

In the holding device 450 constructed as described above, the arm mechanism for supporting the holder 462 has a plurality of rotation axes X1 to X5 and is therefore capable of freely adjusting the position and the angle of the holder 462. Therefore, the holder 462 can be placed with reference to an insertion opening in the patient 1 (the mouth or the anus) and can be set in an insertion direction suitable for the patient 1. If the endoscope 10 or the insertion assist implement 70 is held in the holder 462, it can be smoothly inserted.

In the case of the holding device 450, one of the endoscope 10 and the insertion assist implement 70 is held by being inserted in the holder 462. For example, when the endoscope 10 is moved, the insertion assist implement 70 is held in the holder 462. When the insertion assist implement 70 is moved, the endoscope 10 is held in the holder 462. The holder 462 may be used in common for the endoscope 10 and the insertion assist implement 70.

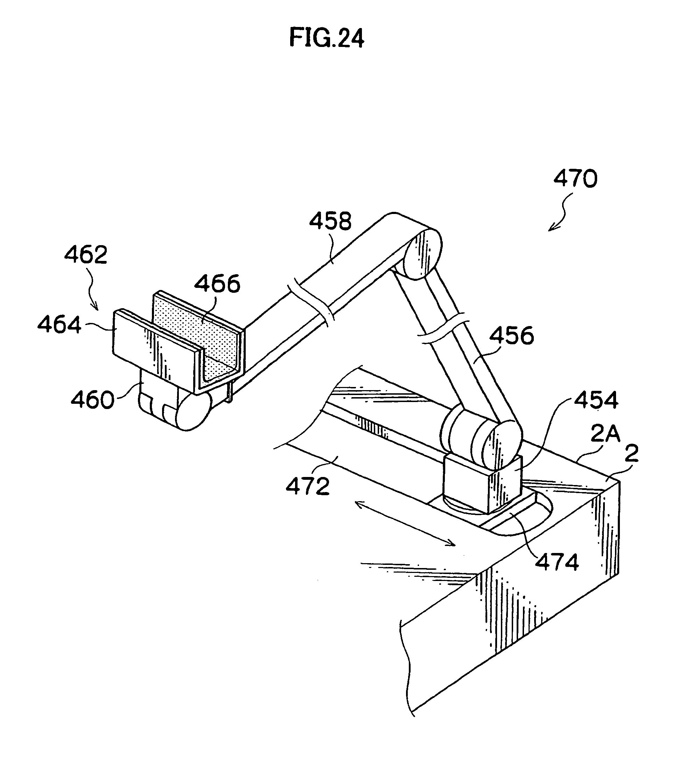

In the above-described embodiment, the holding device 450 is fixed on the examination table 2. The described arrangement, however, is not exclusively used but may be made such that the holding device 450 is movable. For example, a holding device 470 shown in Fig. 24 is constructed so as to be movable along a guide rail 472 formed in the examination table 2. The guide rail 472 is linearly formed along an edge 2A of the examination table 2. A movable base 474 is mounted so as to be movable along the guide rail 472, and the rotary base 454 of the holding device 470 is fixed on the movable base 474. The components above the fixed portion 454 (arms 456 and 458, connecting member 460 and holder 462) are the same as those of the holding device 450 shown in Fig. 23.

The holding device 470 constructed as described above is capable of moving the holder 462 through a wider region since the entire holding device 470 can be moved along the guide rail 472. Also, the holding device 470 is moved along the guide rail 472 while holding the endoscope 10 or the insertion assist implement 70 in the holder 462 so as to move the endoscope 10 or the insertion assist implement 70 in a direction parallel to the guide rail 472. Therefore, an operation to force in or draw out the endoscope 10 or the insertion assist implement 70 can be performed.

The holding device 470 may be moved manually or automatically. The shape of the guide rail 472 is not limited to the linearly-formed shape. The guide rail 472 may be formed into any shape suitable for insertion of the endoscope 10 and the insertion assist implement 70. For example, the guide rail 472 may be formed into a rectangular shape along all the edges of the examination table 2 to enable the holder 462 to be placed at any position in a wide region above the examination table 2.

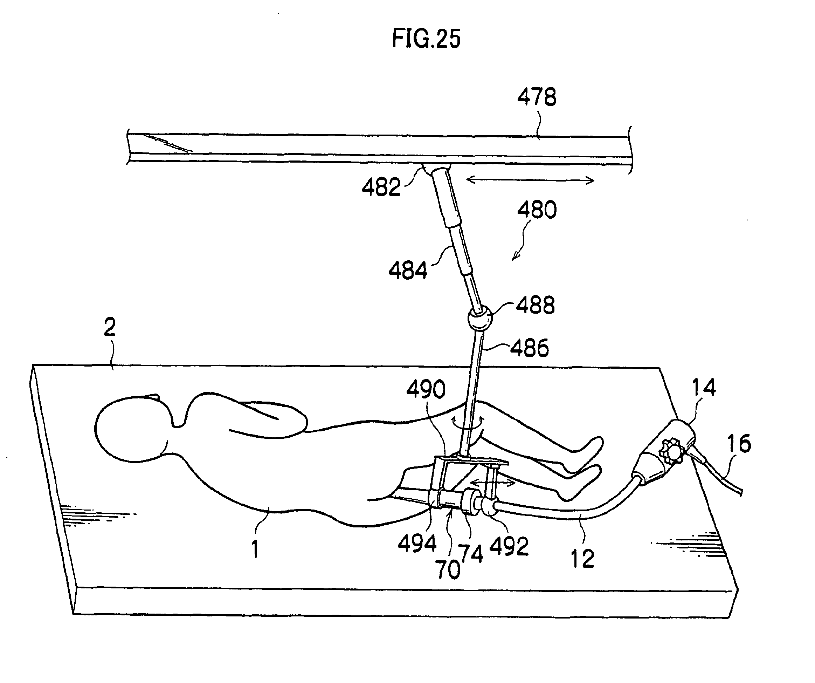

While in the above-described embodiment the guide rail 472 is formed in the examination table 2, the guide rail 472 may be formed on the assist table 3 shown in Fig. 1 or any peripheral unit other than the examination table 2. Further, as shown in Fig. 25, a guide rail 478 may be placed in a ceiling surface above the examination table 2. In the case of the arrangement shown in Fig. 25, a holding device 480 has a movable portion 482 capable of moving along the guide rail 478, and an arm 484 is connected to the movable portion 482 through a universal joint. The arm 484 is constructed so as to be extendible and contractible in a telescopic manner. An arm 486 is connected to the distal end of the arm 484 through a universal joint 488. Two holders 492 and 494 are attached to the lower end of the arm 486 through a distance adjustment device 490. The two holders 492 and 494 are supported by the distance adjustment device 490 so that the distance therebetween can be adjusted by the distance adjustment device 490. Each of the holders 492 and 494 is formed so as to be generally C-shaped. The insertion portion 12 of the endoscope 10 can be held by being fitted in the holder 492. The insertion assist implement 70 can be held by being fitted in the holder 494. The distance adjustment device 490 is rotatably attached to the arm 486.

The holding device 480 constructed as described above can move along the guide rail 478 provided in a ceiling surface and can therefore move the holders 492 and 494 through a wider region and place the holders 492 and 494 at any positions above the examination table 2. The holding device 480 can also retract the holders 492 and 494 to a higher position when the holders 492 and 494 are not used. Further, the holding device 480 can force in or draw out the endoscope 10 and the insertion assist implement 70 while adjusting the distance between the holders 492 and 494 by means of the distance adjustment device 490, and can smoothly insert the endoscope 10 and the insertion assist implement 70 into the patient 1.

In the above-described holding device 480, the operation to move the movable portion 482, the operation to extend or contract the arm 484, the operation to turn the arms 484 and 486 on each end and the operation to adjust the distance between the holders 492 and 494 may be automatically performed by using drive devices such as motors or cylinders.

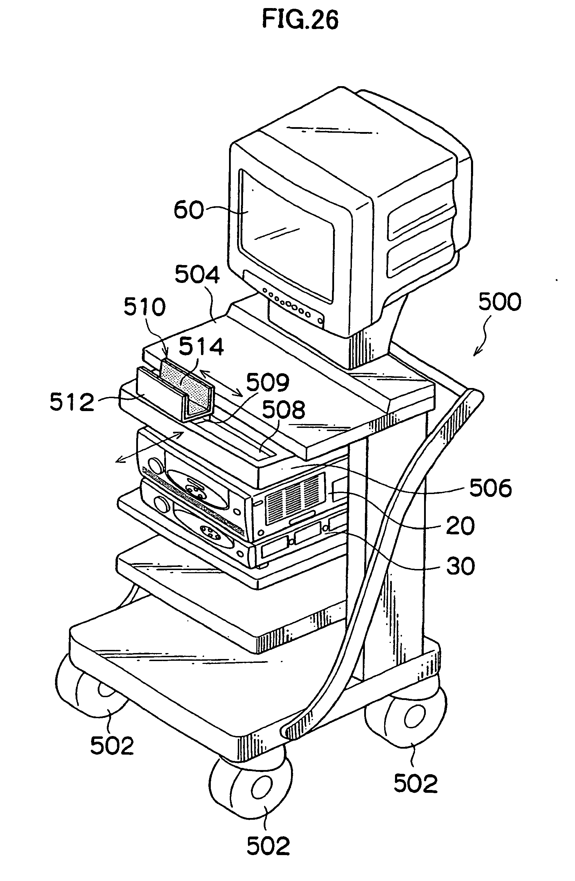

Fig. 26 is a perspective view of another example of the holding device. The holding device shown in Fig. 26 is integrally formed on a cart 500. The cart 500 is a dolly on which the light source unit 20, the processor 30 and the monitor 60 are mounted, which has wheels 502, and which is constructed so as to be freely movable. A lock mechanism (not shown) is attached to the wheels 502 to enable the cart 500 to be fixed. The cart 500 has a fixed table 504, and a movable table 506 capable of being drawn out on the front side. A guide rail 508 is formed in the movable table 506 along a lateral direction (i.e., a direction perpendicular to the direction of movement of the movable table 506). A traveller 509 is mounted on the guide rail 508 so as to be movable along the guide rail 508, and a holder 510 is mounted on the traveler 509. The traveler 509 is automatically moved along the guide rail 508 by a drive device (not shown). The movable table 506 is moved relative to the fixed table 504 by a drive device (not shown). The traveler 509 and the movable table 506 may be respectively moved manually.

The holder 510 is constituted of a supporting member 512 formed so as to be generally C-shaped and an elastic member 514 placed inside the supporting member 512, as is the holder 462 shown in Fig. 24. The holder 510 is capable of holding the at-hand operating portion 14 and the base end portion 74 of the insertion assist implement 70 in a state of being inserted therein.

The cart 500 constructed as described above is moved by rotating the wheels 502 so that the holder 510 is placed in the vicinity of an insertion opening in the patient 1 (the mouth or the anus), and the wheels 502 are locked. The endoscope 10 or the insertion assist implement 70 inserted into the patient 1 is held by being inserted in the holder 512 when necessary. Thus, the need to hold the endoscope 10 or the insertion assist implement 70 is eliminated and one operator can operate the endoscope 10 or the insertion assist implement 70 by him/herself. Adjustment of direction of insertion of the endoscope 10 or the insertion assist implement 70 can be performed by moving the movable table 506. Also, the endoscope 10 or the insertion assist implement 70 held in the holder 510 can be moved in the direction of insertion by moving the traveler 509 along the guide rail 508. Thus, the endoscope 10 or the insertion assist implement 70 can be automatically inserted into the patient 1.

The arrangement of the above-described device may alternatively be such that two holders 510 are provided and each holder 510 is moved along the guide rail 508. The endoscope 10 and the insertion assist implement 70 can be held in the two holders 510 to be respectively moved automatically.

While in the above-described embodiment the holder 510 can be moved along the guide rail 508, the arrangement for moving the holder 510 in this manner is not exclusively used. The holder 510 may be fixed, for example, on the movable table 506 or the fixed table 508. Also in such case, the position of the holder 510 can be adjusted by moving the cart 500. Further, the holder 510 may be detachably attached, for example, to the movable table 506 in a fitting or magnetization manner for example. If the holder 510 is detachably attached in such a manner, the holder 510 can be moved to any position while holding the endoscope 10 or the insertion assist implement 70 with the holder 510. Thus, the holder 510 can be placed in a position suitable for insertion into the patient 1 or can be temporarily put aside to such a position as to avoid interference with examination operations. The holders 210 and 230 shown in Fig. 4, the holders 412 and 422 shown in Fig. 19 or 23, or the holder 462 shown in Fig. 20 may be formed so as to be detachably attached, and may be detached and mounted on the cart 500 or the examination table 2.

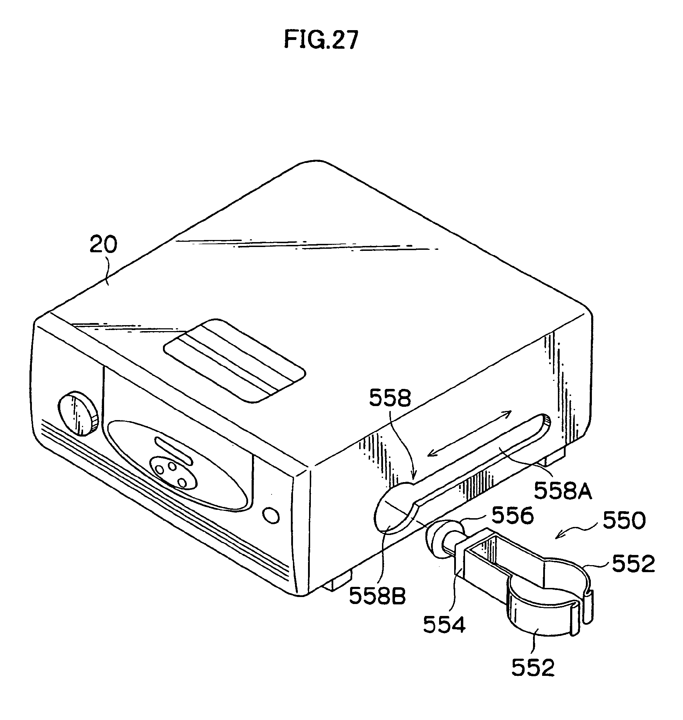

Fig. 27 is a perspective view of another example of the holding device. A holder 550 shown in Fig. 27 has a pair of pinching plates 552 facing each other which are attached to a fixing member 554 while being spaced apart from each other by a predetermined distance. The pair of pinching plates 552 can be elastically deformed outwardly. The endoscope 10 or the insertion assist implement 70 is inserted between the pair of pinching plates 552 to be pinched between the same. Portions of the pinching plates 552 are respectively curved into circular-arc shapes having convex outer surfaces. The endoscope 10 or the insertion assist implement 70 is pinched between the circular-arc portions.

The holder 550 is detachably mounted in a side surface of the light source unit 20 and movably supported on the same. That is, a projection 556 having a generally semispherical shape is provided on the fixing portion 554 of the holder 550. This projection 556 is inserted into an opening 558 in the side surface of the light source unit 20. The opening 558 is formed of a slit opening portion 558A elongated in the direction of the arrow and an attachment opening portion 558B opened largely at one end of the slit opening portion 558A. The projection 556 of the holder 550 is inserted into the attachment opening portion 558B and moved along the slit opening 588A. The holder 550 is thus supported so as to be movable in the direction of the arrow. A fitting member (not shown) into which the projection 556 is fitted is provided in the light source unit 20, and a drive device (not shown) for driving the fitting member in the direction of the arrow is also provided. Therefore, the holder 550 can be automatically moved in the direction of the arrow. The holder 550 may also be moved manually.

Also in the case where the endoscope 10 or the insertion assist implement 70 is held in the holder 550 constructed as described above, the endoscope 10 or the insertion assist implement 70 held in the holder 550 can be moved. As a result, the endoscope 10 or the insertion assist implement 70 can be smoothly inserted.

While an example of mounting the holder 550 in a side surface of the light source unit 20 has been described with reference to Fig. 27, the described arrangement is not exclusively used. For example, the holder 550 may be mounted in a font surface or an upper surface of the light source unit 20. The holder 550 may also be mounted on a peripheral unit, e.g., the processor 30 or the balloon control apparatus 100. Further, the holder 550 may be mounted on the examination table 2 or the like.

While an example of slidably mounting the holder 550 in the direction of the arrow has been described with reference to Fig. 27, the described arrangement is not exclusively used. For example, the holder 550 may be fixed in the light source unit 20 or the like.

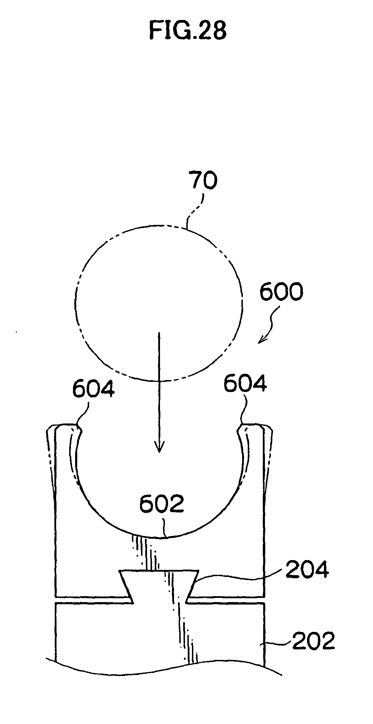

Figs. 28 to 30 show examples of modifications of the assist implement holder 230 shown in Fig. 5. A channel 602'which has a circular-arc shape as viewed in section and in which the insertion assist implement 70 is fitted is formed in an upper surface of a holder 600 shown in Fig. 28, and a pair of pinching portions 504 are formed on opposite sides of the channel 602. The holder 600 is made of a material such as a plastic elastically deformable, and the pair of pinching portions 504 are elastically deformed outwardly. When the insertion assist implement 70 is inserted into the channel 602 of the holder 600 from above, the pair of pinching portions 504 are elastically deformed outwardly to allow the insertion assist implement 70 to be fitted in the channel 602. The original shape of the pair of pinching portions 504 is restored by the resilience. The insertion assist implement 70 is thereby pinched and held between the pair of pinching portions 504.

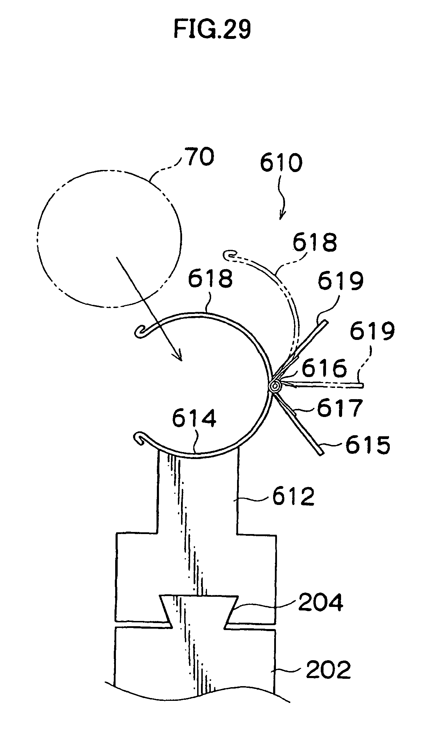

A holder 610 shown in Fig. 29 is constituted of a slidable member 612 supported on the guide rail 204 so as to be slidable on the same, a fixed pinching member 614 fixed on the upper end of the slidable member 612 and having a generally semicircular shape, and a movable pinching member 618 turnably connected to the fixed pinching member 614 by a pin 616 and having a generally semicircular shape. Lugs 615 and 619 are respectively formed integrally with the fixed pinching member 614 and the movable pinching member 618. A spring 617 is mounted between the lugs 615 and 619 to urge the lugs 615 and 619 in such a direction that the angle between the lugs 615 and 619 is increased (that is, the distance between the fixed pinching member 614 and the movable pinching member 618 is reduced). In the holder 610 constructed as described above, the angle between the lugs 615 and 619 is reduced against the urging force of the spring 617 to increase the distance between the fixed pinching member 614 and the movable pinching member 618. The insertion assist implement 70 is then placed between the fixed pinching member 614 and the movable pinching member 618. The lugs 615 and 619 are thereafter released from the hand to pinch the insertion assist implement 70 between the fixed pinching member 614 and the movable pinching member 618 by utilizing the urging force of the spring 617, thereby holding the insertion assist implement 70 in the holder 610.

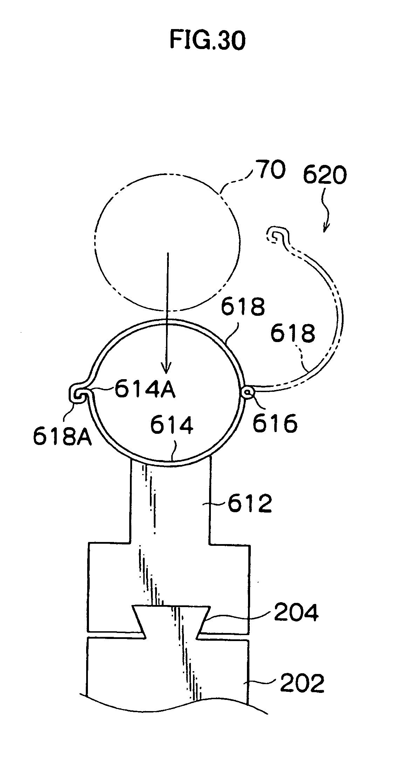

In a holder 620 shown in Fig. 30, opening ends of a fixed pinching member 614 and a movable pinching member 618 are respectively bend to form fitting portions 614A and 618A, which are fitted into each other. When the insertion assist implement 70 is pinched between the fixed pinching member 614 and the movable pinching member 618 in the holder 620, the fitting portions 614A and 618A are fitted into each other to hold the insertion assist implement 70 more firmly.

## Claims

1. A balloon control apparatus which controls operations for expanding and contracting a first balloon (42) attached to an insertion portion (12) of an endoscope (10), and operations for expanding and contracting a second balloon (72) attached to an insertion assist implement (70) fitted around the insertion portion (12) to assist insertion of the insertion portion (12), the apparatus **characterized by** comprising:
a storage device which stores an operating procedure indicating a sequence of the operation for expanding the first balloon (42), the operation for contracting the first balloon (42), the operation for expanding the second balloon (72), and the operation for contracting the second balloon (72);
an execution direction device which directs execution of the expanding and contracting operations according to the operation procedure stored by the storage device;
a plurality of manual operation buttons individually corresponding to the operation for expanding the first balloon (42), the operation for contracting the first balloon (42), the operation for expanding the second balloon (72), and the operation for contracting the second balloon (72);
an automatic operation button corresponding to the execution direction device; and
a mode change switch which selects between a manual mode in which the manual operation buttons are made operable and an automatic mode in which the automatic operation button is made operable,
wherein a direction is given by the execution direction device to execute the operation for expanding the first balloon (42), the operation for contracting the first balloon (42), the operation for expanding the second balloon (72), and the operation for contracting the second balloon (72) in accordance with the sequence indicated by the operating procedure stored by the storage device.

2. The apparatus according to claim 1, wherein the automatic mode is automatically cancelled when one of the manual operation buttons is operated.

3. The apparatus according to claim 1 or claim 2, further comprising: a learning function which stores in the storage device the sequence of operations defined by operation of the manual operation buttons as the operating procedure.

4. The apparatus according to claim 1, wherein
the storage device stores a plurality of operating procedures, and
the apparatus further comprises a selection operation device which selects an operating procedure to be executed from among the plurality of operating procedures.

## Patentansprüche

1. Ballonsteuervorrichtung, die Operationen zum Expandieren und Kontrahieren eines ersten, an einem Einführteil (12) eines Endoskops (10) befestigten Ballons (42) steuert, außerdem Operationen zum Expandieren und Kontrahieren eines zweiten Ballons (72), der an einem Einführhilfsinstrument (70) befestigt ist, welches das Einführteil (12) umgibt, um das Einführen des Einführteils (12) zu unterstützen, **gekennzeichnet durch**:
eine Speichereinrichtung, die eine Operationsprozedur speichert, welche eine Abfolge des Vorgangs zum Expandieren des ersten Ballons (42), des Vorgangs zum Kontrahieren des ersten Ballons (42), des Vorgangs zum Expandieren des zweiten Ballons (72) und des Vorgangs zum Kontrahieren des zweiten Ballons (72) angibt;
eine Ausführungs-Leiteinrichtung, die das Ausführen der Expandier- und Kontrahieroperationen gemäß der von der Speichereinrichtung gespeicherten Operationsprozedur leitet;
eine Mehrzahl von manuellen Betätigungsknöpfen, die individuell dem Vorgang zum Expandieren des ersten Ballons (42), dem Vorgang zum Kontrahieren des ersten Ballons (42), dem Vorgang zum Expandieren des zweiten Ballons (72) und dem Vorgang zum Kontrahieren des zweiten Ballons (72) entsprechen;
einen Automatikbetätigungsknopf entsprechend der Ausführungs-Leiteinrichtung; und
einen Modus-Änderungsschalter, der auswählt zwischen einem manuellen Modus, bei dem die manuellen Betätigungsknöpfe betätigbar sind, und einem Automatikmodus, bei dem der Automatikbetätigungsknopf betätigbar ist,
wobei von der Ausführungs-Leiteinrichtung eine Anweisung zum Ausführen des Vorgangs zum Expandieren des ersten Ballons (42), des Vorgangs zum Kontrahieren des ersten Ballons (42), des Vorgangs zum Expandieren des zweiten Ballons (72) und des Vorgangs zum Kontrahieren des zweiten Ballons (72) nach Maßgabe der Folge gegeben wird, die **durch** die von der Speichereinrichtung gespeicherte Operationsprozedur vorgegeben ist.

2. Vorrichtung nach Anspruch 1, bei der der Automatikmodus automatisch aufgehoben wird, wenn einer der manuellen Betätigungsknöpfe betätigt wird.

3. Vorrichtung nach Anspruch 1 oder 2, weiterhin umfassend:
eine Lernfunktion, die in der Speichereinrichtung als Operationsprozedur die Folge von Operationen speichert, die durch die Betätigung der manuellen Betätigungsknöpfe definiert wird.

4. Vorrichtung nach Anspruch 1, bei der die Speichereinrichtung eine Mehrzahl von Operationsprozeduren speichert, und die Vorrichtung außerdem eine Auswahleinrichtung aufweist, die eine Operationsprozedur auswählt, welche von den mehreren Operationsprozeduren ausgeführt werden soll.

## Revendications

1. Appareil de commande de ballonnet qui commande les opérations de dilatation et de contraction d'un premier ballonnet (42) fixé à une partie d'introduction (12) d'un endoscope (10), et les opérations de dilatation et de contraction d'un second ballonnet (72) fixé à un instrument d'aide à l'introduction (70) ajusté autour de la partie d'introduction (12) pour aider à l'introduction de la partie d'introduction (12), l'appareil étant **caractérisé en ce qu'**il comprend :
un dispositif de stockage qui stocke une procédure d'actionnement indiquant une séquence de l'opération de dilatation du premier ballonnet (42), de l'opération de contraction du premier ballonnet (42), de l'opération de dilatation du second ballonnet (72), et de l'opération de contraction du second ballonnet (72) ;
un dispositif de direction d'exécution qui dirige l'exécution des opérations de dilatation et de contraction en fonction de la procédure d'actionnement stockée par le dispositif de stockage ;
une pluralité de boutons d'actionnement manuel correspondant individuellement à l'opération de dilatation du premier ballonnet (42), à l'opération de contraction du premier ballonnet (42), à l'opération de dilatation du second ballonnet (72), et à l'opération de contraction du second ballonnet (72) ;
un bouton d'actionnement automatique correspondant au dispositif de direction d'exécution ; et
un commutateur de changement de mode qui sélectionne un mode manuel dans lequel les boutons d'actionnement manuel sont actionnables ou un mode automatique dans lequel le bouton d'actionnement automatique est actionnable,
dans lequel une direction est donnée par le dispositif de direction d'exécution pour exécuter l'opération de dilatation du premier ballonnet (42), l'opération de contraction du premier ballonnet (42), l'opération de dilatation du second ballonnet (72), et l'opération de contraction du second ballonnet (72) conformément à la séquence indiquée par la procédure d'actionnement stockée par le dispositif de stockage.

2. Appareil selon la revendication 1, dans lequel le mode automatique est automatiquement annulé lorsque l'un des boutons d'actionnement manuel est actionné.

3. Appareil selon la revendication 1 ou la revendication 2, comprenant en outre : une fonction d'apprentissage qui stocke dans le dispositif de stockage la séquence d'opérations définie par l'actionnement des boutons d'actionnement manuel comme procédure d'actionnement.

4. Appareil selon la revendication 1, dans lequel
le dispositif de stockage stocke une pluralité de procédures d'actionnement, et
l'appareil comprend en outre un dispositif d'opération de sélection qui sélectionne une procédure d'actionnement à exécuter parmi la pluralité de procédures d'actionnement.
